(19) Europäisches Patentamt
European Patent Office
Office européen des brevets

(11) EP 3 782 977 A1

(12) **EUROPEAN PATENT APPLICATION**

(43) Date of publication:
**24.02.2021 Bulletin 2021/08**

(51) Int Cl.:
***C07C 67/313*** *(2006.01)* ***C07C 69/743*** *(2006.01)*

(21) Application number: **19382720.1**

(22) Date of filing: **22.08.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Fundació Privada Institut Català d'Investigació Química (ICIQ)**
**43007 Tarragona (ES)**

(72) Inventors:
- **García Suero, Marcos**
  **43005 Tarragona (ES)**
- **García Herraiz, Ana**
  **16700 Sisena (ES)**

# (54) CYCLOPROPANATION METHOD AND REAGENT

(57) The present invention relates to a metal-free photochemical method for the preparation of cyclopropanes from substrates comprising one carbon-carbon double bond conjugated with an aromatic or heteroaromatic group which advantageously takes place with no need for a photosensitizer. It also relates to certain reagents useful in said transformation.

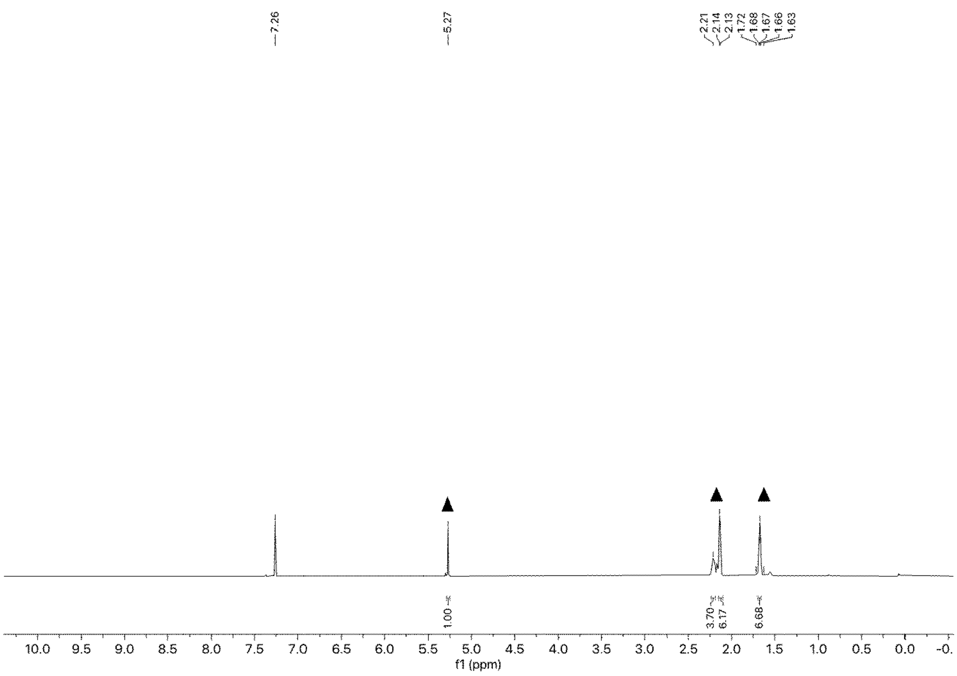


FIGURE 1

EP 3 782 977 A1

## Description

The present invention relates to a metal-free photochemical method for the preparation of cyclopropanes from substrates comprising one carbon-carbon double bond conjugated with an aromatic or heteroaromatic group which advantageously takes place with no need for a photosensitizer. It also relates to certain reagents useful in said transformation.

## BACKGROUND ART

The cyclopropyl group is a common building block in several fine chemicals, drugs, and drug candidates as it is reported in the art to improve in certain cases the therapeutic profile of the drug candidate. In particular, cyclopropyl residues adjacent to aromatic or heteroaromatic groups are common scaffolds in drug candidates and can be found for instance in the anti-depressive drugs tranylcypromine, milnacipran and levomilnacipran as well as in drugs for the treatment of sleep disorders such as tasimelteon or the anti-hypercholesterolaemia drug pitavastatin. Cyclopropyl groups are also very common in fragrance ingredients such as jasminol®, toscanol®, pashminol® as they are reported to boost the flavouring properties of the ingredient. The chemical community thus developed several methods and processes for the preparation of cyclopropanes. A common strategy in that sense relies upon the reaction of carbenes or carbenoids with carbon-carbon double bonds of the substrate.

Cyclopropanation methods based on the reaction of an alkene with a carbene or carbenoid are known in the art. As carbenes are unstable chemical species, it is quite common to rely on the use of carbenoids to generate the carbene in situ. In the Simmons-Smith reaction, diiodomethane is reacted with zinc and copper in stoichiometric amounts to produce iodomethylzinc iodide which attacks the double bond of the olefin substrate to form the cyclopropane. This method requires the use of large amounts of metal. Alternative procedures based on the use of diazo compounds are known in the art, each of which suffers from safety hazards related to the use of explosive diazo reagents. Such procedures may be based either on the metal-free cycloaddition of the diazo compound to the double bond, leading to a pyrazoline ring that undergoes denitrogenation to form the cyclopropane moiety (Kischner reaction), or on the use of a metal-based catalyst (such as rhodium) to form the metal-carbene species that reacts with the double bond.

Diiodomethyl compounds have further been investigated as potential carbenoids for the development of cyclopropanation methods. Del Hoyo and co-workers (Angew. Chem. Int. Ed. 2017, 56, 1610 -1613.) have reported the use of diiodomethane as a carbenoid precursor using photocatalytic conditions. The developed method comprises contacting a styrene compound with diiodomethane under light irradiation in the presence of a stoichiometric amount of a tertiary amine as electron donor. In this method, the use of a catalytic amount of a ruthenium-based photocatalyst is further indicated as providing high yields in the cyclopropanation of styrene-like derivatives. Also, according to the authors, the addition of sodium thiosulfate in water to the reaction medium may further be used to prevent the inhibition of the photosensitizer by undesired iodine by-products and thus increase the yield of reaction. As further reported by the same authors (Eur. J. Org. Chem. 2017, 2122-2125.), similar reaction conditions provided cyclopropanated products when the substrate of the reaction is a 1,4-unsaturated ketone or aldehyde.

Diiodomethylcompounds substituted with an ester group have further been investigated by Li and co-workers as cyclopropanation reagents (Nature Communications, 2018, 9:1972 - DOI: 10.1038/s41467-018-04331-4.). These compounds are formed within the reaction mixture by reaction between a diazoacetate compound and a catalytic amount of iodine. The reported cyclopropanation method consists in contacting a styrene-like substrate with a diazoacetate compound under irradiation with light and in the presence of catalytic amounts of iodine and a photosensitizer. Both the iodine and the photosensitizer are reported as being essential for the reaction to proceed. When using the pre-formed diiodomethyl ester compound in the conditions of reaction, no cyclopropanation is observed under the reported conditions. It is also reported that the cyclopropanation proceeds with a moderate yield when a pre-formed diiodomethyl ester compound is used for the cyclopropanation of a styrene-like substrate under the conditions reported by Del Hoyo and co-workers.

Sayes and co-workers have described the use of diiodomethyl compound substituted with a boronate group as carbenoid precursors in the photochemical borocyclopropanation of styrene compounds carried out under continuous flow (Angew. Chem. Int. Ed. 2018, 57, 13514 -13518.). The reported method consists in feeding a continuous flow reactor irradiated with light with a solution comprising the styrene-like substrate, a diiodomethyl boronate compound, and a tertiary amine as electron donor. As previously observed by Del Hoyo and co-workers, the addition of a catalytic amount of a chromophore or photosensitizer, such as xanthone, to the reaction mixture is beneficial as it allows increasing substantially the yield of reaction.

An alternative cyclopropanation method using diiodomethyl silylated reagents was reported by Phelan and co-workers (J. Am. Chem. Soc. 2018, 140, 8037-8047.). The cyclopropanation method consists in contacting a styrene-like substrate with the pre-formed diiodomethyl silylated reagent under irradiation of blue light and in the presence of a catalytic amount of a chromophore photosensitizer. According to the authors, the photosensitizer is an essential feature

to allow for the cyclopropanation to occur.

From what is known in the art, it derives that there is still a need for providing improved methods and reagents for the preparation of cyclopropanated compounds from diiodomethyl reagents that provide high yields of cyclopropanated product with no need for a photosensitizer to be used.

## SUMMARY OF THE INVENTION

The inventors after extensive and exhaustive research have developed a new method for the preparation of cyclopropanated compounds from olefins wherein the double bond is adjacent to an aromatic or heteroaromatic group, said method comprising the step of contacting the olefin compound with a diiodomethyl carbonyl compound under light irradiation in the presence of an electron donor and of a biphasic solvent system consisting of an organic and an aqueous layers. The inventors found that, in these conditions of reaction, the yield of the cyclopropanated product is surprisingly high, even when a photosensitizer is not used in the reaction mixture. This is unexpected insofar as the photosensitizer is reported to be beneficial to the outcome of the reaction according to the prior art related to cyclopropanation methods using diiodomethyl carbenoid compounds as cyclopropanation reagents. In addition, in view of the prior art, it is a rather unexpected technical effect that an increase in the yields of the cyclopropanated product is obtained when the reaction is carried out in a biphasic system. The inventors have also found a diiodomethyl carbonyl compound useful in the cyclopropanation method of the invention that can advantageously be stored at room temperature. This presents the further technical advantage of using shelf-stable reagents. While most diiodomethyl carbonyl compounds described in the prior art suffer from poor stability to light and temperature and tend to decompose quickly, the use of shelf-stable reagents provides a higher usability of the cyclopropanation method of the invention which can be carried out without having to prepare the carbenoid diiodomethyl compound shortly before the reaction or in situ.

Thus, in a first aspect, the present invention relates to a method for the preparation of a compound comprising a fragment of formula (I) from a compound comprising a fragment of formula (II)

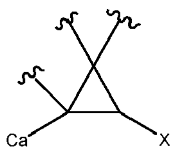

(I)

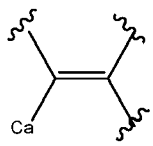

(II)

wherein the method converts the fragment of formula (II) into the fragment of formula (I), $C_a$ is a carbon atom that is member of an aromatic or heteroaromatic ring system, each wavy line represents a connection to the remaining parts of the compound comprising the fragment of formula (I) or of formula (II), X is a radical selected from the group consisting of a radical of formula $-COR_1$, and a radical of formula $-COOR_1$, being $R_1$ a radical selected from the group consisting of hydrogen, a $(C_1-C_{12})$alkyl radical a benzyl radical and a radical deriving from a ring system comprising from 1 to 4 bridged or fused rings, each ring comprising from 3 to 6 members and being saturated or aromatic, the members of the rings being selected from the group consisting of C, CH, $CH_2$, O, S, N and NH and wherein each ring is further optionally substituted at any available position with a radical selected from the group consisting of nitro, halo, cyano, $(C_1-C_{12})$alkyl, $(C_1-C_{12})$haloalkyl, $(C_1-C_{12})$alkyloxy, $(C_1-C_{12})$alkylcarbonyl, and $(C_1-C_{12})$alkyloxycarbonyl and $(C_1-C_{12})$alkylcarbonyloxy, said method comprising the step of contacting the compound comprising the fragment of formula (II) with a compound of formula (III)

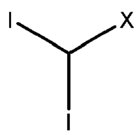

(III)

under light irradiation and in the presence of an electron donor and of a biphasic solvent system consisting of an organic phase and an aqueous phase.

The invention also provides shelf-stable compounds useful as reagents in the method of the invention. A second aspect of the invention therefore relates to a compound of formula (III')

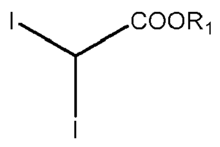

(III')

that is stable in its isolated form, wherein $R_1$ is as defined in the first aspect of the invention and the term "stable in its isolated form" refers to the fact that the purity of the isolated compound does not decrease at a rate higher than one percent per day upon storage under air, at room temperature and ambient light irradiation.

In a third aspect, the invention relates to the use of a compound according to the second aspect of the invention as a reagent for cyclopropanation reactions.

## BRIEF DESCRIPTION OF THE DRAWINGS

Figure 1 shows a $^1H$ NMR spectrum of the compound of formula (IIIa) (which is a compound of formula (III') wherein $R_1$ is adamantyl) as obtained after synthesis and purification. Black triangles show the peaks assigned to the compound of formula (IIIa).

Figure 2 shows a $^1H$ NMR spectrum of an equimolar mixture of the compound of formula (IIIa) as obtained after 40 days of storage under air, at room temperature and under ambient light irradiation with 1,3,5-trimethoxybenzene as internal standard. Black triangles show the peaks assigned to the compound of formula (IIIa), while asterisks show the peaks assigned to 1,3,5-trimethoxybenzene.

Figure 3 shows a $^1H$ NMR spectrum of an equimolar mixture of the compound of formula (IIIa) as obtained after 40 days of storage under air, at room temperature and in the dark with 1,3,5-trimethoxybenzene as internal standard. Black triangles show the peaks assigned to the compound of formula (IIIa), while asterisks show the peaks assigned to 1,3,5-trimethoxybenzene.

Figure 4 shows a $^1H$ NMR spectrum of an equimolar mixture of the compound of formula (IIIa) as obtained after 40 days of storage under argon atmosphere, at room temperature and in the dark with 1,3,5-trimethoxybenzene as internal standard. Black triangles show the peaks assigned to the compound of formula (IIIa), while asterisks show the peaks assigned to 1,3,5-trimethoxybenzene.

## DETAILED DESCRIPTION OF THE INVENTION

All terms as used herein in this application, unless otherwise stated, shall be understood in their ordinary meaning as known in the state of the art. Other more specific definitions for certain terms as used in the present application are as set forth below and are intended to apply uniformly throughout the specification and claims, unless an otherwise expressly set out definition provides a broader definition.

For the purposes of the present invention, any ranges given include both the lower and the upper end-points of the range. Ranges given, such as temperatures, times, molar ratio and the like, should be considered approximate (this is, with a 5% margin of variation around indicated point), unless specifically stated otherwise.

In the context of the present invention, the term "aromatic ring system" refers to a molecular fragment consisting of a conjugated planar ring system made of carbon atoms wherein the number of delocalized electrons is 4m+2, being m an integer number, being said system connected to the remainder parts of the compound comprising an aromatic ring system through any available position. Aromatic ring systems known in the art include, for instance, benzene, naphthalene, phenanthrene, anthracene and tetracene.

In the context of the present invention, the term "heteroaromatic ring system" refers to a molecular fragment consisting of a conjugated planar ring system made of carbon atoms or heteroatoms such as O, S, NH and N wherein the number of delocalized electrons is 4m+2, being m an integer number, being said system connected to the remainder parts of the compound comprising an aromatic ring system through any available position. Heteroaromatic ring systems known in the art include, for instance, furane, thiophene, pyridine, pyrazine, pyrimidine, pyrrole, imidazole, pyrazole, oxazole, isoxazole, thiazole, pyridazine, triazine, benzofuran, isobenzofuran, indole, isoindole, benzothiophene, benzo[c] thiophene, benzimidazole, purine, indazole, benzoxazole, benzisoxazole, benzothiazole, quinoline, isoquinoline, quinoxaline, acridine, quinazoline, cinnoline, and phthalazine.

In the context of the present invention, the term $(C_1-C_{12})$alkyl refers to a saturated, linear or branched, hydrocarbon chain having from 1 to 12 carbon atoms. Non-limiting examples of $(C_1-C_{12})$alkyl groups include methyl, ethyl,

propyl, *iso*-propyl, n-butyl, *tert*-butyl, *iso*-butyl, *sec*-butyl, *n*-pentyl, *tert*-pentyl, *iso*-pentyl, *sec*-pentyl, *neo*pentyl, 1-ethylpropyl, *n*-hexyl, 1-methyl-pentyl, 2-methyl-pentyl, 3-methyl-pentyl, 4-methyl-pentyl, 3,3-dimethylbutyl, 2,2-dimethylbutyl, 1,1-dimethylbutyl, 2,3-dimethylbutyl, 1,3-dimethylbutyl,1,2-dimethylbutyl, 3-ethylbutyl, 2-ethylbutyl, 1-ethylbutyl, *n*-heptyl, *n*-octyl, *n*-nonyl, n-decanyl, undecanyl, and dodecanyl.

In the context of the present invention, the term ($C_2$-$C_{12}$)alkenyl refers to a saturated, linear or branched, hydrocarbon chain having from 2 to 12 carbon atoms and further comprising a carbon-carbon double bond.

In the context of the present invention, the term ($C_2$-$C_{12}$)alkynyl refers to a saturated, linear or branched, hydrocarbon chain having from 2 to 12 carbon atoms and further comprising a carbon-carbon triple bond.

In the context of the present invention, the term ($C_1$-$C_{12}$)haloalkyl refers to a saturated, linear or branched, hydrocarbon chain having from 1 to 12 carbon atoms wherein one or more of the hydrogen atoms are replaced by a halo group (chloro, fluoro, bromo or iodo). Non-limiting examples of ($C_1$-$C_{12}$)haloalkyl groups include chloromethyl, bromomethyl, fluoromethyl, difluoromethyl, trifluoromethyl. Preferred embodiments of ($C_1$-$C_{12}$)haloalkyl are those wherein one or more of the hydrogen atoms are replaced by a chloro, bromo or fluoro group.

In the context of the present invention, the term ($C_1$-$C_{12}$)alkyloxy refers to a saturated, linear or branched, hydrocarbon chain radical having from 1 to 12 carbon atoms that is appended to the remainder of the molecule through an oxygen (O) atom. Non-limiting examples of ($C_1$-$C_{12}$)alkyloxy groups include methoxy, ethoxy, propyloxy, *iso*-propyloxy, *n*-butyloxy, *tert*-butyloxy, *iso*-butyloxy, *sec*-butyloxy, *n*-pentyloxy, *tert*-pentyloxy, *iso*-pentyloxy, *sec*-pentyloxy, *neo*-pentyloxy, 1-ethylpropyloxy, *n*-hexyloxy, 1-methyl-pentyloxy, 2-methyl-pentyloxy, 3-methyl-pentyloxy, 4-methyl-pentyloxy, 3,3-dimethylbutyloxy, 2,2-dimethylbutyloxy, 1,1-dimethylbutyloxy, 2,3-dimethylbutyloxy, 1,3-dimethylbutyloxy,1,2-dimethylbutyloxy, 3-ethylbutyloxy, 2-ethylbutyloxy, 1-ethylbutyloxy, *n*-heptyloxy, *n*-octyloxy, *n*-nonyloxy, *n*-decanyloxy, undecanyloxy, and dodecanyloxy.

In the context of the present invention, the term ($C_1$-$C_{12}$)alkyloxycarbonyl refers to a saturated, linear or branched, hydrocarbon chain radical having from 1 to 12 carbon atoms that is appended to the remainder of the molecule through an oxycarbonyl (-O(C=O)-) group.

In the context of the present invention, the term ($C_1$-$C_{12}$)alkylcarbonyl refers to a saturated, linear or branched, hydrocarbon chain radical having from 1 to 12 carbon atoms that is appended to the remainder of the molecule through a carbonyl (C=O) group.

In the context of the present invention, the term ($C_1$-$C_{12}$)alkylcarbonyloxy refers to a saturated, linear or branched, hydrocarbon chain radical having from 1 to 12 carbon atoms that is appended to the remainder of the molecule through an oxycarbonyl (-(C=O)O-) group.

In the context of the present invention, within a ring system comprising at least two rings, two rings are said to be "bridged" when they have at least two atoms in common in their cyclic structure, said atoms not being adjacent in either cyclic ring.

In the context of the present invention, within a ring system comprising at least two rings, two rings are said to be "fused" when they have at least two atoms in common in their cyclic structure, said atoms being adjacent in the cyclic rings.

In the context of the present invention, the term "shelf-stable" or "stable in its isolated form" refers to the fact that the purity of the isolated compound does not decrease at a rate higher than one percent per day upon storage under air, at room temperature and ambient light irradiation. Suitable means are known in the art to measure the purity of an organic compound, using analytical techniques such as liquid chromatography, $^1$H NMR spectroscopy (with or without internal standards), and UV-Vis spectrometry.

As already noted above, according to a first aspect, the present invention refers to a photochemical cyclopropanation method as described above, which comprises the step of contacting a compound comprising a fragment of formula (II) as described above with a compound of formula (III)

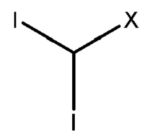

(III)

under light irradiation and in the presence of an electron donor and an aqueous solution of sodium chloride.

In preferred embodiments of the first aspect, the invention relates to a method for the preparation of a compound comprising a fragment of formula (I) from a compound comprising a fragment of formula (II) that is carried out under an inert atmosphere. In particular, it is further preferred that the method of the invention is carried out in a non-oxidant atmosphere. A suitable atmosphere to carry out the method of the invention is an oxygen-free atmosphere, preferably an atmosphere of an inert gas, and more preferably an atmosphere of nitrogen or argon.

The method of the first aspect of the invention is highly chemoselective. Unlike other cyclopropanation methods described in the state of the art using olefin starting materials, and as illustrated in the Examples section, the method of the invention selectively forms the cyclopropanated product at the carbon-carbon double bond that is adjacent to an aromatic or heteroaromatic group. It is advantageous since it saves the steps of protecting and unprotecting additional double bonds present on the reaction substrate. The method of the first aspect of the invention is also robust as it tolerates the presence of functional groups present in the compound comprising the fragment of formula (II).

In preferred embodiments of the first aspect, the invention relates to a method for the preparation of a compound comprising a fragment of formula (I) from a compound comprising a fragment of formula (II), wherein $C_a$ is a carbon atom that is member of a molecular fragment selected from the group consisting of benzene, naphthalene, phenanthrene, anthracene, tetracene furane, thiophene, pyridine, pyrazine, pyrimidine, pyrrole, imidazole, pyrazole, oxazole, isoxazole, thiazole, pyridazine, triazine, benzofuran, isobenzofuran, indole, isoindole, benzothiophene, benzo[c]thiophene, benzimidazole, purine, indazole, benzoxazole, benzisoxazole, benzothiazole, quinoline, isoquinoline, quinoxaline, acridine, quinazoline, cinnoline, and phthalazine, and wherein said molecular fragment is connected to the remainder parts of the compound comprising a fragment of formula (II) at any available position.

In further preferred embodiments of the first aspect, the invention relates to a method for the preparation of a compound comprising a fragment of formula (I) from a compound comprising a fragment of formula (II), wherein $C_a$ is a carbon atom that is member of a molecular fragment selected from the group consisting of benzene, benzofuran, pyrazole, indole, benzothiophene and thiophene, and wherein said molecular fragment is connected to the remainder parts of the compound comprising a fragment of formula (II) at any available position.

In further preferred embodiments of the first aspect, the invention relates to a method for the preparation of a compound comprising a fragment of formula (I) from a compound comprising a fragment of formula (II), wherein $C_a$ is a carbon atom member of an aromatic or heteroaromatic ring system as described in any of the above embodiments and further optionally substituted at any available position with a radical selected from the group consisting of $(C_1\text{-}C_{12})$alkyl, $(C_2\text{-}C_{12})$alkenyl, $(C_2\text{-}C_{12})$alkynyl, nitro, cyano, halo, $(C_1\text{-}C_{12})$haloalkyl, $(C_1\text{-}C_{12})$alkyloxy, $(C_2\text{-}C_{12})$alkenyloxy, $(C_2\text{-}C_{12})$alkynyloxy, $(C_1\text{-}C_{12})$alkylthioxy, $(C_2\text{-}C_{12})$alkenylthioxy, $(C_2\text{-}C_{12})$alkynylthioxy, $(C_1\text{-}C_{12})$alkylcarbonyl, $(C_1\text{-}C_{12})$alkyloxycarbonyl, $(C_1\text{-}C_{12})$alkylcarbonyloxy, $C_1\text{-}C_{12})$alkylcarbonylamino, $C_1\text{-}C_{12})$alkylaminocarbonyl, a formyl group, a carboxy group, $(C_1\text{-}C_{12})$alkylboronate, pinacol boronate, glucosyl, fructosyl and a ring system comprising from 1 to 3 rings, said rings comprising from 3 to 7 members selected from the group consisting of C, CH, CO, $CH_2$, N, NH, O and S, the rings being saturated, unsaturated or aromatic, isolated, bridged or fused and being further optionally substituted at any available position with a radical selected from the group consisting of $(C_1\text{-}C_{12})$alkyl, $(C_2\text{-}C_{12})$alkenyl, $(C_2\text{-}C_{12})$alkynyl, nitro, cyano, halo, $(C_1\text{-}C_{12})$haloalkyl, $(C_1\text{-}C_{12})$alkyloxy, $(C_2\text{-}C_{12})$alkenyloxy, $(C_2\text{-}C_{12})$alkynyloxy, $(C_1\text{-}C_{12})$alkylthioxy, $(C_2\text{-}C_{12})$alkenylthioxy, $(C_2\text{-}C_{12})$alkynylthioxy, $(C_1\text{-}C_{12})$alkylcarbonyl, $(C_1\text{-}C_{12})$alkyloxycarbonyl, $(C_1\text{-}C_{12})$alkylcarbonyloxy, a formyl group, a carboxy group, $(C_1\text{-}C_{12})$alkylboronate, pinacol boronate, glucosyl, and fructosyl.

In certain preferred embodiments of the first aspect, the invention relates to a method for the preparation of a compound comprising a fragment of formula (I) from a compound comprising a fragment of formula (II), wherein $C_a$ is a carbon atom that is member of a molecular fragment selected from the group consisting of benzene, benzofuran, pyrazole, indole, benzothiophene and thiophene, said molecular fragment being further optionally substituted at one or more available position with a radical selected from the group consisting of $(C_1\text{-}C_{12})$alkyl, $(C_2\text{-}C_{12})$alkenyl, $(C_2\text{-}C_{12})$alkynyl, halo, $(C_1\text{-}C_{12})$alkyloxy, $(C_2\text{-}C_{12})$alkenyloxy, $(C_2\text{-}C_{12})$alkynyloxy, $(C_1\text{-}C_{12})$alkylthioxy, $(C_1\text{-}C_{12})$alkyloxycarbonyl, $(C_1\text{-}C_{12})$alkylcarbonylamino, a formyl group, a carboxy group, a pinacol boronate, glucosyl and fructosyl.

In other preferred embodiments of the first aspect, the invention relates to a method for the preparation of a compound comprising a fragment of formula (I) from a compound comprising a fragment of formula (II), wherein $C_a$ is a carbon atom member of an aromatic or heteroaromatic ring system that is bridged or fused with a further ring system comprising from 1 to 3 rings, said rings comprising from 3 to 7 members selected from the group consisting of C, CH, CO, $CH_2$, N, NH, O and S, the rings being saturated, unsaturated or aromatic, isolated, bridged or fused and being further optionally substituted at any available position with a radical selected from the group consisting of $(C_1\text{-}C_{12})$alkyl, $(C_2\text{-}C_{12})$alkenyl, $(C_2\text{-}C_{12})$alkynyl, nitro, cyano, halo, $(C_1\text{-}C_{12})$haloalkyl, $(C_1\text{-}C_{12})$alkyloxy, $(C_2\text{-}C_{12})$alkenyloxy, $(C_2\text{-}C_{12})$alkynyloxy, $(C_1\text{-}C_{12})$alkylthioxy, $(C_2\text{-}C_{12})$alkenylthioxy, $(C_2\text{-}C_{12})$alkynylthioxy, $(C_1\text{-}C_{12})$alkylcarbonyl, $(C_1\text{-}C_{12})$alkyloxycarbonyl, $(C_1\text{-}C_{12})$alkylcarbonyloxy, a formyl group, a carboxy group, $(C_1\text{-}C_{12})$alkylboronate, pinacol boronate, glucosyl, and fructosyl.

In further preferred embodiments of the first aspect, the invention relates to a method for the preparation of a compound comprising a fragment of formula (I) from a compound comprising a fragment of formula (II), wherein the fragment of formula (II) is a fragment of formula (II')

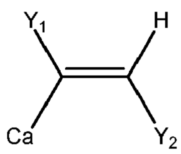

(II')

wherein $Y_1$ and $Y_2$ independently represent a hydrogen atom or a carbon atom through which the molecular fragment of formula (II) is connected to the remainder parts of the compound comprising the fragment of formula (II).

In further preferred embodiments, embodiments of the first aspect, the invention relates to a method for the preparation of a compound comprising a fragment of formula (I) from a compound comprising a fragment of formula (II), wherein the fragment of formula (II) is a fragment of formula (II') as defined above wherein $Y_1$ and $Y_2$ are each independently selected from the group consisting of $(C_1$-$C_{12})$alkyl, $(C_2$-$C_{12})$alkenyl, $(C_2$-$C_{12})$alkynyl and a radical of formula Z, being each of the $(C_1$-$C_{12})$alkyl, $(C_2$-$C_{12})$alkenyl, $(C_2$-$C_{12})$alkynyl group further optionally substituted with a radical selected from the group consisting of hydroxyl, amino, formyl, carboxyl and a radical of formula Z; being Z a radical deriving from a ring system comprising from 1 to 3 rings, said rings comprising from 3 to 7 members selected from the group consisting of C, CH, CO, $CH_2$, N, NH, O and S, the rings being saturated, unsaturated or aromatic, isolated, bridged or fused and being further optionally substituted at any available position with a radical selected from the group consisting of $(C_1$-$C_{12})$alkyl, $(C_2$-$C_{12})$alkenyl, $(C_2$-$C_{12})$alkynyl, nitro, cyano, halo, $(C_1$-$C_{12})$haloalkyl, $(C_1$-$C_{12})$alkyloxy, $(C_2$-$C_{12})$alkenyloxy, $(C_2$-$C_{12})$alkynyloxy, $(C_1$-$C_{12})$alkylthioxy, $(C_2$-$C_{12})$alkenylthioxy, $(C_2$-$C_{12})$alkynylthioxy, $(C_1$-$C_{12})$alkylcarbonyl, $(C_1$-$C_{12})$alkyloxycarbonyl, $(C_1$-$C_{12})$alkylcarbonyloxy, a formyl group, a carboxy group, $(C_1$-$C_{12})$alkylboronate, pinacol boronate, glucosyl, and fructosyl; or, alternatively, one or two of the pairs $Y_1$ and $Y_2$, $Y_1$ and a member of the aromatic or heteroaromatic ring system comprising $C_a$, $Y_2$ and a member of the aromatic or heteroaromatic ring system comprising $C_a$, form a ring system comprising from 1 to 3 rings, said rings comprising from 3 to 7 members selected from the group consisting of C, CH, CO, $CH_2$, N, NH, O and S, the rings being saturated, unsaturated or aromatic, isolated, bridged or fused and being further optionally substituted at any available position with a radical selected from the group consisting of $(C_1$-$C_{12})$alkyl, $(C_2$-$C_{12})$alkenyl, $(C_2$-$C_{12})$alkynyl, nitro, cyano, halo, $(C_1$-$C_{12})$haloalkyl, $(C_1$-$C_{12})$alkyloxy, $(C_2$-$C_{12})$alkenyloxy, $(C_2$-$C_{12})$alkynyloxy, $(C_1$-$C_{12})$alkylthioxy, $(C_2$-$C_{12})$alkenylthioxy, $(C_2$-$C_{12})$alkynylthioxy, $(C_1$-$C_{12})$alkylcarbonyl, $(C_1$-$C_{12})$alkyloxycarbonyl, $(C_1$-$C_{12})$alkylcarbonyloxy, a formyl group, a carboxy group, $(C_1$-$C_{12})$alkylboronate, pinacol boronate, glucosyl, and fructosyl.

In further preferred embodiments of the first aspect, the invention relates to a method for the preparation of a compound comprising a fragment of formula (I) from a compound comprising a fragment of formula (II), wherein the fragment of formula (II) is a fragment of formula (II') as defined above wherein $Y_1$ and $Y_2$ are each independently selected from the group consisting of $(C_1$-$C_{12})$alkyl, $(C_2$-$C_{12})$alkenyl, and a radical of formula Z, being each of the $(C_2$-$C_{12})$alkenyl, $(C_2$-$C_{12})$alkynyl group further optionally substituted with a radical selected from the group consisting of hydroxyl, and a radical of formula Z, wherein Z is a radical deriving from a ring system comprising from 1 to 3 rings, said rings comprising from 4 to 6 members selected from the group consisting of C, CH, CO, $CH_2$, N, NH, O and S, the rings being saturated, unsaturated or aromatic, isolated, bridged or fused; or, alternatively, one or two of the pairs $Y_1$ and $Y_2$, $Y_1$ and a member of the aromatic or heteroaromatic ring system comprising $C_a$, $Y_2$ and a member of the aromatic or heteroaromatic ring system comprising $C_a$, form a hydrocarbon saturated ring, said rings comprising from 5 to 6 members.

In other preferred embodiments of the first aspect, the invention relates to a method for the preparation of a compound comprising a fragment of formula (I) from a compound comprising a fragment of formula (II) as defined above wherein the electron donor is selected from the group consisting of a cathode, a photo-cathode and a compound of formula $NR_2R_3R_4$ wherein each of $R_2$, $R_3$ and $R_4$ is a hydrogen or a $(C_1$-$C_6)$alkyl group, equal or different.

In other preferred embodiments of the first aspect, the invention relates to a method for the preparation of a compound comprising a fragment of formula (I) from a compound comprising a fragment of formula (II) as defined above wherein the electron donor is selected from the group consisting of a cathode, a photo-cathode and a compound of formula $NR_2R_3R_4$ wherein each of $R_2$, $R_3$ and $R_4$ is a $(C_1$-$C_6)$alkyl group, equal or different. In further preferred embodiments of the first aspect, the invention relates to a method for the preparation of a compound comprising a fragment of formula (I) from a compound comprising a fragment of formula (II) as defined above wherein the electron donor is a compound of formula $NR_2R_3R_4$ wherein each of $R_2$, $R_3$ and $R_4$ is a $(C_1$-$C_6)$alkyl group, equal or different.

In further preferred embodiments of the first aspect, the invention relates to a method for the preparation of a compound comprising a fragment of formula (I) from a compound comprising a fragment of formula (II) as defined above wherein the electron donor is selected from the group consisting of trimethylamine, triethylamine and diisopropylethylamine. More preferably, in such embodiments, the electron donor is diisopropylethylamine.

In further preferred embodiments of the first aspect, the invention relates to a method for the preparation of a compound comprising a fragment of formula (I) from a compound comprising a fragment of formula (II) as defined above

wherein when the electron donor is a compound of formula $NR_2R_3R_4$ wherein each of $R_2$, $R_3$ and $R_4$ is a ($C_1$-$C_6$)alkyl group, equal or different, the molar ratio of the compound of formula $NR_2R_3R_4$ to the compound comprising the fragment of formula (II) is at least 2n:n, being n the number of fragments of formula (II) in the compound comprising the fragment of formula (II). It is further preferred that when the electron donor is a compound of formula $NR_2R_3R_4$ wherein each of $R_2$, $R_3$ and $R_4$ is a ($C_1$-$C_6$)alkyl group, equal or different, the molar ratio of the compound of formula $NR_2R_3R_4$ to the compound comprising the fragment of formula (II) is 5n:n. For example, when the compound comprising a fragment of formula (II) comprises one fragment of formula (II), n is 1 and the molar ratio of the compound of formula $NR_2R_3R_4$ to the compound comprising the fragment of formula (II) is preferably of 5:1. It is further preferred that n is 1.

In further preferred embodiments of the first aspect, the invention relates to a method for the preparation of a compound comprising a fragment of formula (I) from a compound comprising a fragment of formula (II) as defined above wherein the electron donor is diisopropylethylamine and the molar ratio of diisopropylethylamine to the compound comprising the fragment of formula (II) is at least 2n:n, being n the number of fragments of formula (II) in the compound comprising the fragment of formula (II). Preferably, the electron donor is diisopropylethylamine and the molar ratio of diisopropylethylamine to the compound comprising the fragment of formula (II) is 5n:n, being n the number of fragments of formula (II) in the compound comprising the fragment of formula (II).

In other preferred embodiments of the first aspect, the invention relates to a method for the preparation of a compound comprising a fragment of formula (I) from a compound comprising a fragment of formula (II) as defined above wherein the biphasic solvent system consisting of an organic phase and an aqueous phase is such that the organic phase is a solvent selected from the group consisting of acetonitrile, tetrahydrofuran, 1,4-dioxane, diethyl ether, methyl-*tert*-butylether, dichloromethane, dichloroethane, chloroform and tetrachloroethane, and the aqueous phase is selected from water and an aqueous solution of a water soluble salt in a suitable concentration for the solvent system to be biphasic. In further preferred embodiments, the organic phase of the biphasic solvent system is a solvent selected from the group consisting of acetonitrile, tetrahydrofuran and dichloromethane. In further preferred embodiments, the aqueous phase of the biphasic solvent system is water when the organic phase is a solvent that is non-miscible with water. In additional further preferred embodiments, the aqueous phase of the biphasic solvent system is an aqueous solution of a water soluble salt when the organic phase is a solvent that is miscible with water. Suitable water soluble salts for the purposes of the method of the fist aspect of the invention are those that do not substantially prevent the formation of the compound comprising the moiety a formula (I). Water soluble salts having non-oxidant properties are preferred. Water soluble salts having non-acidic or non-basic properties are preferred. In specific embodiments, the aqueous phase of the biphasic solvent system is an aqueous solution of a water soluble salt when the organic phase is a solvent that is miscible with water wherein the water soluble salt is selected from the group consisting of alkaline or alkaline earth salts of halide, nitrate and sulfate. In more preferred specific embodiments, the aqueous phase of the biphasic solvent system is an aqueous solution of a water soluble salt when the organic phase is a solvent that is miscible with water wherein the water soluble salt is selected from the group consisting of sodium chloride, sodium bromide, and lithium chloride. More preferably, the aqueous phase of the biphasic solvent system is an aqueous solution of a water soluble salt when the organic phase is a solvent that is miscible with water wherein the water soluble salt is sodium chloride.

In other preferred embodiments of the first aspect, the invention relates to a method for the preparation of a compound comprising a fragment of formula (I) from a compound comprising a fragment of formula (II) as defined above wherein the biphasic solvent system consists of an organic phase that is acetonitrile and of an aqueous phase that is an aqueous solution of a sodium chloride in a suitable concentration for the solvent system to be biphasic.

A suitable concentration for the solvent system to be biphasic when the organic phase is acetonitrile and the water soluble salt is sodium chloride is comprised in the range of 0.5 to 5 moles per liter. It is preferred that the concentration for the solvent system to be biphasic when the organic phase is acetonitrile and the water soluble salt is sodium chloride is comprised in the range of 1 to 2 moles per liter; even more preferably, it is of 1.25 mole per liter.

Without being bound to theory, it is believed that the presence of a biphasic system comprising an aqueous phase is essential to the production of the compound comprising a moiety of formula (I) as it allows the removal from the organic phase of light-absorbing iodine-deriving by-products which would otherwise inhibit the formation of the reaction product if present in the organic phase. The use of a biphasic system is thus advantageous as it allows for the production of the compound comprising a moiety of formula (I) in high yields when no photosensitizer is used.

In other preferred embodiments of the first aspect, the invention relates to a method for the preparation of a compound comprising a fragment of formula (I) from a compound comprising a fragment of formula (II) as defined above that is carried out under light irradiation having a wavelength in the range of 350 to 800 nm. Suitable means to apply a wavelength in the range of 350 to 800 nm are known in the art and include compact fluorescent lamps (CFL), blue light emitting diodes (LEDs) and white LEDs. Therefore, in preferred embodiments, the method of the first aspect of the invention is carried out under irradiation of light emitted from a source selected from the group consisting of compact fluorescent lamps (CFL), blue light emitting diodes (LEDs) and white LEDs. In further preferred embodiments, the method of the first aspect of the invention is carried out under light irradiation emitted from a compact fluorescent lamp. When the method of the first aspect of the invention is carried out under irradiation of a CFL, it is further preferred that the

potency of irradiation is of at least 15 W, more preferably of at least 20 W. In certain preferred embodiments, when the method of the first aspect of the invention is carried out under irradiation of a CFL the potency of irradiation is of 21 W.

In other preferred embodiments of the first aspect, the invention relates to a method for the preparation of a compound comprising a fragment of formula (I) from a compound comprising a fragment of formula (II) as defined above wherein the molar ratio of the compound of formula (III) to the compound comprising the fragment of formula (II) is comprised from n:n to 3n:n, being n the number of fragments of formula (II) in the compound comprising the fragment of formula (II). For example, when the compound comprising a fragment of formula (II) comprises one fragment of formula (II), n is 1 and the molar ratio of the compound of formula (III) to the compound comprising the fragment of formula (II) is comprised from 1:1 to 3:1. It is further preferred that n is 1.

In further preferred embodiments of the first aspect, the invention relates to a method for the preparation of a compound comprising a fragment of formula (I) from a compound comprising a fragment of formula (II) as defined above wherein the molar ratio of the compound of formula (III) to the compound comprising the fragment of formula (II) is 2n:n, being n the number of fragments of formula (II) in the compound comprising the fragment of formula (II). For example, when the compound comprising a fragment of formula (II) comprises one fragment of formula (II), n is 1 and the molar ratio of the compound of formula (III) to the compound comprising the fragment of formula (II) is preferably 2:1.

In other preferred embodiments of the first aspect of the invention, the invention relates to a method for the preparation of a compound comprising a fragment of formula (I) from a compound comprising a fragment of formula (II) as defined above wherein, in the compound of formula (III), X is a radical selected from the group consisting of a radical of formula $-COR_1$ and a radical of formula $-COOR_1$, being $R_1$ a radical selected from the group consisting of a $(C_1-C_{12})$alkyl radical, a benzyl radical and a radical deriving from a ring system comprising from 1 to 4 bridged or fused rings, each ring comprising from 3 to 6 members and being saturated or aromatic, the members of the rings being selected from the group consisting of C, CH, $CH_2$, O, S, N and NH and wherein each ring is further optionally substituted at any available position with a radical selected from the group consisting of nitro, halo, cyano, $(C_1-C_{12})$haloalkyl, $(C_1-C_{12})$alkyloxy, $(C_1-C_{12})$alkylcarbonyl, and $(C_1-C_{12})$alkyloxycarbonyl and $(C_1-C_{12})$alkylcarbonyloxy.

In other preferred embodiments of the first aspect of the invention, the invention relates to a method for the preparation of a compound comprising a fragment of formula (I) from a compound comprising a fragment of formula (II) as defined above wherein, in the compound of formula (III), X is a radical selected from the group consisting of a radical of formula $-COR_1$ and a radical of formula $-COOR_1$, being $R_1$ a radical selected from the group consisting of a $(C_1-C_{12})$alkyl radical, a benzyl radical and a radical deriving from a ring system comprising from 1 to 4 bridged or fused rings, each ring comprising from 3 to 6 members and being saturated or aromatic, the members of the rings being selected from the group consisting of C, CH, and $CH_2$, and wherein each ring is further optionally substituted at any available position with a radical selected from the group consisting of nitro, halo, cyano, $(C_1-C_{12})$haloalkyl, $(C_1-C_{12})$alkyloxy, $(C_1-C_{12})$alkylcarbonyl, and $(C_1-C_{12})$alkyloxycarbonyl and $(C_1-C_{12})$alkylcarbonyloxy.

In further preferred embodiments of the first aspect of the invention, the invention relates to a method for the preparation of a compound comprising a fragment of formula (I) from a compound comprising a fragment of formula (II) as defined above wherein, in the compound of formula (III), X is selected from the group consisting of a radical of formula $-COR_1$, and a radical of formula $-COOR_1$ being $R_1$ a radical selected from the group consisting of a $(C_1-C_{12})$alkyl radical, a benzyl radical, a $(C_6-C_{20})$aryl radical and a radical deriving from a saturated hydrocarbon ring system comprising from 1 to 4 bridged or fused rings, each ring comprising from 3 to 6 members.

In further preferred embodiments of the first aspect of the invention, the invention relates to a method for the preparation of a compound comprising a fragment of formula (I) from a compound comprising a fragment of formula (II) as defined above wherein, in the compound of formula (III), X is selected from the group consisting of a radical of formula $-COR_1$, and a radical of formula $-COOR_1$ being $R_1$ a radical selected from the group consisting of a methyl, ethyl, *iso*-propyl, propyl, *tert*-butyl, butyl, benzyl, phenyl, naphthyl, cyclohexyl, bicyclohexyl, and adamantyl.

In further preferred embodiments of the first aspect of the invention, the invention relates to a method for the preparation of a compound comprising a fragment of formula (I) from a compound comprising a fragment of formula (II) as defined above wherein, in the compound of formula (III), X is selected from the group consisting of a radical of formula $-COR_1$, and a radical of formula $-COOR_1$ being $R_1$ selected from the group consisting of ethyl, phenyl, adamantyl, butyl, benzyl and *tert*-butyl.

In further preferred embodiments of the first aspect of the invention, the invention relates to a method for the preparation of a compound comprising a fragment of formula (I) from a compound comprising a fragment of formula (II) as defined above wherein, in the compound of formula (III), X is selected from the group consisting of butylcarbonyl, *tert*-butylcarbonyl, phenylcarbonyl, ethylcarbonyloxy, benzylcarbonyloxy and adamantylcarbonyloxy.

In more preferred embodiments of the first aspect, , the invention relates to a method for the preparation of a compound comprising a fragment of formula (I) from a compound comprising a fragment of formula (II) as defined above wherein two, three or four of the following options are combined:

(i) X is selected from the group consisting of a radical of formula $-COR_1$, and a radical of formula $-COOR_1$ being $R_1$

a radical selected from the group consisting of a ($C_1$-$C_{12}$)alkyl radical, a benzyl radical, a ($C_6$-$C_{20}$)aryl radical and a radical deriving from a saturated hydrocarbon ring system comprising from 1 to 4 bridged or fused rings, each ring comprising from 3 to 6 members;
(ii) the method of the first aspect of the invention is carried out under irradiation of light emitted from a source selected from the group consisting of compact fluorescent lamps (CFL), blue light emitting diodes (LEDs) and white LEDs;
(iii) the electron donor is a compound of formula $NR_2R_3R_4$ wherein each of $R_2$, $R_3$ and $R_4$ is a ($C_1$-$C_6$)alkyl group, equal or different;
(iv) the biphasic solvent system consists of an organic phase that is acetonitrile and of an aqueous phase that is an aqueous solution of a sodium chloride in a suitable concentration for the solvent system to be biphasic.

In even more preferred embodiments of the first aspect, , the invention relates to a method for the preparation of a compound comprising a fragment of formula (I) from a compound comprising a fragment of formula (II) as defined above wherein two, three, four or five of the following options are combined:

(i) X is selected from the group consisting of a radical of formula -$COR_1$, and a radical of formula -$COOR_1$ being $R_1$ a radical selected from the group consisting of a methyl, ethyl, iso-propyl, propyl, *tert*-butyl, butyl, phenyl, benzyl, naphthyl, cyclohexyl, bicyclohexyl, and adamantyl;
(ii) the method of the first aspect of the invention is carried out under irradiation of light emitted from a source a compact fluorescent lamp (CFL);
(iii) the electron donor is diisopropylethylamine;
(iv) the biphasic solvent system consists of an organic phase that is acetonitrile and of an aqueous phase that is an aqueous solution of a sodium chloride in a suitable concentration for the solvent system to be biphasic;
(v) the method is carried out under an inert atmosphere.

It is also further preferred that the method of the invention is carried out at room temperature, that is a temperature comprised from 20 °C to 25 °C.

As will become apparent to the skilled person, when X is a radical of formula - $COOR_1$ as defined in any of the above embodiments, the method of the first aspect of the invention may further comprise the step of converting the radical of formula - $COOR_1$ into a radical of formula -COOH. Suitable methods for the hydrolysis of esters, such as the group of formula -$COOR_1$, are known in the art and are common general knowledge.

The method of the first aspect of the invention is advantageous as it allows producing compounds comprising a fragment of formula (I) in high yields with no need for the use of a photosensitizer. The method of the invention is further highly chemoselective, to the extent that only double bonds adjacent to aromatic or heteroaromatic groups are converted into cyclopropyl groups, which advantageously affords saving several synthetic steps of protection/deprotection of additional double bonds present on the compound comprising the fragment of formula (I).

According to the second aspect, the invention relates to a compound of formula (III') as defined above that is stable in its isolated form, and wherein the term "stable in its isolated form" refers to the fact that the purity of the isolated compound does not decrease at a rate higher than one percent per day upon storage under air, at room temperature and ambient light irradiation. When the compound of formula (III) is stable in its isolated form, it is a compound of formula (III'). The stability of the compound of formula (III) is advantageous as it allows storing the compound of formula (III) prior to its use in the method of the invention and saves the prior step of preparing the compound of formula (III). Inventors have found that, when in the compound of formula (III), $R_1$ is methyl or ethyl, the compound of formula (III) is not stable in its isolated form. Such compounds are out of the scope of the second aspect of the invention.

In preferred embodiments of the second aspect, the invention relates to a compound of formula (III') as defined above that is stable in its isolated form, and wherein the term "stable in its isolated form" refers to the fact that the purity of the isolated compound does not decrease at a rate higher than 0.5% per day upon storage under air, at room temperature and ambient light irradiation.

In further preferred embodiments of the second aspect, the invention relates to a compound of formula (III') as defined above that is stable in its isolated form, and wherein the term "stable in its isolated form" refers to the fact that the purity of the isolated compound does not decrease at a rate higher than 0.1% per day upon storage under air, at room temperature and ambient light irradiation.

In further preferred embodiments of the second aspect, the invention relates to a compound of formula (III') as defined above that is stable in its isolated form, and wherein the term "stable in its isolated form" refers to the fact that the purity of the isolated compound does not decrease at a rate higher than 0.01% per day upon storage under air, at room temperature and ambient light irradiation.

In other preferred embodiments of the second aspect, the invention relates to a compound of formula (III') as defined above wherein $R_1$ is as defined in any embodiment of the first aspect of the invention and is such that the compound of formula (III') is stable in its isolated form.

In further preferred embodiments of the second aspect, the invention relates to a compound of formula (III') as defined above that is a solid in standard conditions of pressure and temperature. When the compound of formula (III') is a solid in standard conditions of pressure and temperature, it is advantageous as it is easier to handle and weight than liquid compounds.

In further preferred embodiments of the second aspect, the invention relates to a compound of formula (III') as defined above wherein $R_1$ is adamantyl, that is a compound of formula (IIIa)

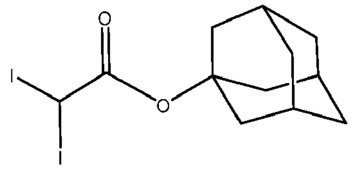

(IIIa)

The compound of formula (IIIa) is stable in its isolated form and is a solid under standard conditions of pressure and temperature. The compound of formula (IIIa) is thus suitable as a shelf-stable reagent for the method of the first aspect of the invention and makes its implementation easier. As mentioned above, using common general knowledge, the skilled person may identify procedures allowing for the removal of the adamantyl group from the compound comprising the fragment of formula (I) wherein X is adamantoyl.

A process for the preparation of a compound of formula (III) is also part of the invention. Such process comprises the step of contacting a compound of formula (IV)

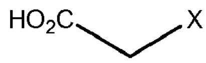

(IV)

wherein X is as defined in any of the embodiments of the first aspect of the invention, with an iodinating agent and in the presence of a base.

In the process of preparation of the compound of formula (III) described above, it is preferred that the iodinating agent is in an amount equal to or higher than twice the amount of the compound of formula (IV).

In the process of preparation of the compound of formula (III) described above, it is preferred that the base is in amount equal to or higher than the amount of the compound of formula (IV).

In the process of preparation of the compound of formula (III) described above, it is preferred that the iodinating agent is N-iodosuccinimide; preferably in an amount of twice the amount of the compound of formula (IV).

In the process of preparation of the compound of formula (III) described above, it is preferred that the base is sodium acetate; preferably in an amount such that the molar ratio of the base to the compound of formula (IV) is 3:2.

The process of preparation of the compound of formula (III) described above is preferably carried out in a polar aprotic solvent, more preferably in acetonitrile.

The process of preparation of the compound of formula (III) described above is preferably carried out at a temperature comprised in the range of 50°C to 100°C. More preferably, it is carried out at a temperature of about 82°C, in particular when the solvent is acetonitrile.

As defined above, the third aspect of the invention relates to the use of a compound according to any of the embodiments of the second aspect of the invention as a reagent for cyclopropanation reactions. The method according to any of the embodiments of the first aspect of the invention is suitable to carry out the use of the third aspect of the invention.

Throughout the description and claims the word "comprises" and variations of the word, are not intended to exclude other technical features, additives, components or steps. Furthermore, the word "comprise" encompasses the case of "consisting of". Additional objects, advantages and features of the present invention will become apparent to those skilled in the art upon examination of the description or may be learned by practice of the present invention. The following examples are provided by way of illustration, and they are not intended to be limiting of the present invention. Furthermore, the present invention covers all possible combinations of particular and preferred embodiments described herein.

## EXAMPLES

General considerations

All reagents were used as purchased and used without further purification. (E)-anethole was purchased from Aldrich and used without further purification. Unless otherwise stated, reactions were carried out under argon atmosphere. Anhydrous solvents (DMF, DMSO, THF) were dried by passing through an activated alumina column on a PureSolv™ solvent purification system (Innovative Technologies, Inc., MA). $CH_3CN$ (Panreac, HPLC grade) and EtOH (Scharlau, anaylitical solvent) were used without any further purification. Blue LED strips (Leroy Merlin Ref. 15379721), white LED strips (Leroy Merlin Ref. 16640246) and compact fluorescent lamp (CFL) (Leroy Merlin Ref. 14640402) were used as visible light sources. Analytical thin layer chromatography was carried out using TLC-aluminum sheets with 0.2 mm of silica gel (Merck GF234). Visualization was achieved using ultraviolet light (254 nm) and chemical staining with vanillin or basic potassium permanganate solutions as appropriate. Flash column chromatography was performed on silica gel (Aldrich, 230-400 mesh). Organic solutions were concentrated under reduced pressure on a Buchi rotatory evaporator. NMR spectra were recorded at 298 K (unless otherwise stated) on a Bruker Avance 300, Bruker Avance 400 Ultrashield and Bruker Avance 500 Ultrashield apparatuses. Chemical shifts ($\delta$) are quoted in ppm relative to residual solvent and coupling constants (J) are quoted in hertz (Hz). Multiplicity is reported with the following abbreviations: s = singlet, d = doublet, t = triplet, q = quartet, dt = doublet of triplets, td = triplet of doublets, ddd=doublet of doublet of doublets. Melting points were measured using open glass capillaries in a Buchi B540 apparatus. Infrared spectra were recorded on a Bruker Tensor 27. Mass spectra were recorded on a Waters LCT Premier spectrometer.

Preparative example 1: Preparation of styrene derivatives

Styrene derivatives were prepared using the Wittig reaction and following general procedures A or B.

General Procedure A: Potassium *tert*-butoxide (1.0 M solution in THF, 16.1 mL, 16.1 mmol) was added to a suspension of the corresponding phosphonium salt (6.46 mmol) in THF (6.5 mL) at 0 °C. After this, the resulting reaction mixture was stirred for 30 min at room temperature. Then, the corresponding aldehyde or ketone (6.46 mmol) was added and stirred until complete conversion (the reaction was monitored by TLC). The mixture was quenched with saturated $NH_4Cl$ aqueous solution (10 mL) and extracted with AcOEt (3 x 10 mL). The combined organic extracts were washed with brine and dried over anhydrous $Na_2SO_4$ and concentrated in vacuo. The crude residues were purified by flash column chromatography on silica gel (AcOEt/hexane mixtures) affording the corresponding alkenes.

General Procedure B: To a solution of the corresponding triphenylphosphonium bromide salt (1.2 eq, 7.7 mmol) in dry THF (0.3 M) at -78 °C, n-Butyllithium (1.6 M in hexanes, 1.2 eq, 5.25 ml) was added dropwise. The temperature of the reaction mixture was allowed to rise to 0 °C for 30 min and then the corresponding aldehyde or ketone (1.0 eq, 7.0 mmol) dissolved in THF (7 ml) was added dropwise over 20 min. The reaction mixture was stirred for 20 min at 0 °C and then allowed to warm up to room temperature and stirred until complete conversion (the reaction was monitored by TLC). The mixture was quenched with saturated aqueous NH4Cl (10 mL) and extracted with AcOEt (3 x 10 mL). The combined organic extracts were washed with brine and dried over anhydrous $Na_2SO_4$ and concentrated in vacuo. The crude residues were purified by flash column chromatography on silica gel (AcOEt/hexane mixtures) affording the corresponding alkenes.

The following styrene compounds were prepared using the phosphonium salt:

Table 1

| Phosphonium salt | Ketone/aldehyde | General procedure | Styrene compound |
|---|---|---|---|
| $[EtP(Ph)_3]^+Br^-$ | N-(4-formylphenyl)-acetamide | A | 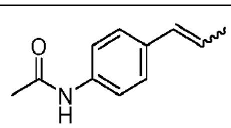 |
| | Undec-10-enal | A | 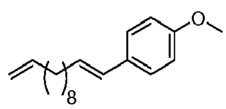 |
| | 4-methoxybenzaldehyde | A | 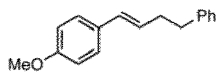 |

(continued)

| Phosphonium salt | Ketone/aldehyde | General procedure | Styrene compound |
|---|---|---|---|
| $[MeP(Ph)_3]^+Br^-$ | 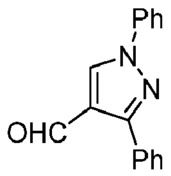 | B | 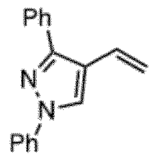 |
| $[MeP(Ph)_3]^+Br^-$ | 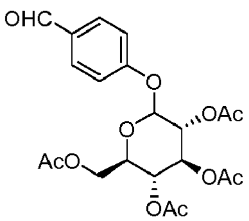 | B | 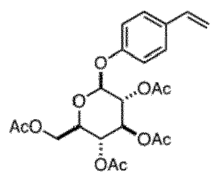 |

Preparative example 2: Preparation of (2R,3S,4S,5R,6S)-2-(hydroxymethyl)-6-(4-vinvlphenoxy)tetrahydro-2H-pyran-3,4,5-triol

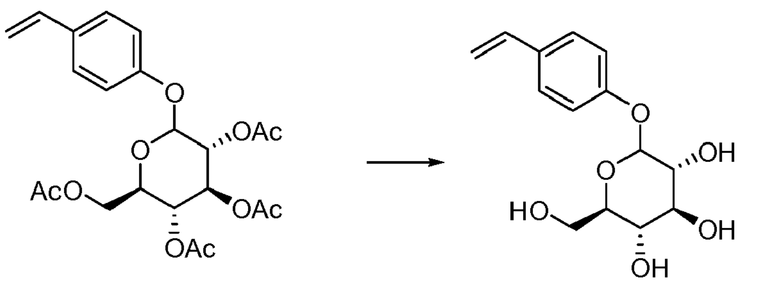

To a round bottom flask was added (2R,3R,4S,5R,6S)-2-(acetoxymethyl)-6-(4-vinylphenoxy)tetrahydro-2H-pyran-3,4,5-triyl triacetate (650 mg, 1.44 mmol, 1 equiv.) and $K_2CO_3$ (2.40 g, 17.0 mmol, 12 equiv.). MeOH (15 ml) was added and the reaction mixture was stirred for 1h at room temperature. Then, a NaOH solution (0.1M, 30ml) was added and the aqueous phase was extracted with AcOEt (3 x 50 ml). The combined organic layers were washed with brine and dried over $Na_2SO_4$. After concentration under vacuo, the corresponding white solid (370 mg, 91% yield) was used without further purification.
m.p. 168-172 °C.

Preparative example 3: Preparation of 2-Methoxy-5-vinylbenzaldehyde

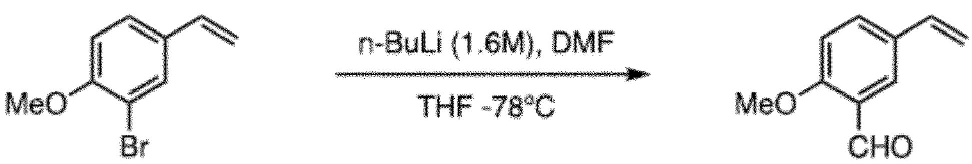


To a solution of 2-bromo-1-methoxy-4-vinylbenzene1 (470 mg, 2.20 mmol) in THF (8.1 mL) at -78 °C, n-butyllithium (1.6 M in hexanes, 1.6 mL, 2.60 mmol) was added dropwise for 10 min and the resulting reaction mixture was stirred for 15 min. Then, N,Ndimethylformamide (1.7 mL, 22.0 mmol) was added at the same temperature, and the mixture was stirred for 2 h. The reaction mixture was quenched with saturated aqueous NH4Cl and extracted with AcOEt (3 x 10 mL). The organic layers were combined, dried (Na2SO4) and concentrated in vacuo. Purification by flash chromatography on silica gel (hexane/AcOEt, 20:1) provided the corresponding aldehyde (230 mg, 65% yield) as a

colourless oil.

Example 1: Preparation of the compound of formula (III) where X is a radical of formula $CO_2R_1$

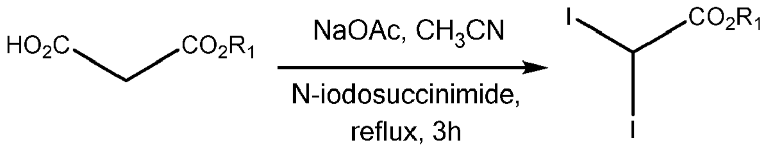


General Procedure C: To a round bottom flask with a stir bar was added N-iodosuccinimide (NIS) (20 mmol, 2 equiv), NaOAc (15 mmol, 1.5 equiv) and the corresponding malonate monoester (10 mmol, 1 equiv). The reaction was degassed and filled with argon (x 3) and MeCN (80 ml, 0.12M) was added. A condenser was placed and the reaction mixture was stirred under reflux. The reaction was monitored by TLC and it was quenched upon complete consumption of the malonate monoester (2-4 h). A $Na_2S_2O3$ saturated aqueous solution (50 ml) was added and extracted with $Et_2O$ (3 x 50 ml). Combined organic layers where dried over $MgSO_4$ and concentrated. The residue was purified by flash chromatography on silica gel (hexane 100% to hexane/EtOAc 50:1). Once the product was eluted from the column, the corresponding fractions were concentrated. If the test tubes were left in the bench the gem-diiodo reagent will slowly decompose and a pick color will appear ($I_2$ formation). In this case, a spatula of $Na_2S_2O_3$ could be added to the collected tubes and stirred until the pink color disappeared, the solid was then removed by filtration and the desired product was obtained after concentration.

Adamantan-1-yl 2,2-diiodoacetate (IIIa)

Prepared according to General Procedure C using 3-((adamantan-1-yl)oxy)-3-oxopropanoic acid (10.3 g, 43 mmol, 1 equiv). Purification by flash chromatography on silica gel (gradient: hexane/EtOAc 100:0 to 100:1) provided the corresponding product (12 g, 65%) as a white pale solid. m.p. 88-91 °C.
IR: 2907, 2850, 1713, 1246, 1120, 1048.
$^1H$ NMR (300 MHz, $CDCl_3$) 5 5.27 (s, 1H), 2.20 (m, 3H), 2.13 (m, 6H), 1.68 (m, 6H). $^{13}C$ NMR (126 MHz, $CDCl_3$) $\delta$ 164.91, 84.27, 40.71, 36.16, 31.03, -40.50.
HRMS (ESI) calculated for $C_{12}H_{16}I_2NaO_2$ [M+Na]+ m/z: 468.9132, found: 468.9120.

Ethyl 2,2-diiodoacetate (IIIb)

Prepared according to General Procedure C using 3-ethoxy-3-oxopropanoic acid (1.2 ml, 0.2 mmol, 1 equiv). Purification by flash chromatography on silica gel (gradient: hexane:EtOAc 100:0 to 100:1) provided the corresponding product (40-65% yield) that was kept in a MeCN solution (1.0-0.5M). Compound was kept in solution in MeCN (1.0-0.5M) at -30°C under argon atmosphere.
IR: 2921, 2850, 1718, 1247, 1118, 1022, 858.
$^1H$ NMR (300 MHz, $CDCl_3$) $\delta$ 5.34 (s, 1 H), 4.28 (q, J = 7.1 Hz, 2H), 1.31 (t, J = 7.1 Hz, 3H).

General procedure for the assessment of stability of a compound of formula (III) in its isolated form

Step 1: A $^1H$ NMR spectrum of the compound of formula (III) in its isolated form is recorded immediately after its preparation, using 1,2-dichloroethane as internal standard, in such a way that the molar ratio of the compound of formula (III) to the internal standard is 1:1. Value of integration of the peak assigned to the proton of the diiodomethyl radical is noted as A1.

Step 2: A certain amount of a compound of formula (III) is stored in its isolated form (i.e. undissolved) in a vial at room temperature, under air and under ambient light irradiation conditions for a certain period of time, counted in days, being I the number of days.

Step 3: A $^1H$ NMR spectrum of the compound of formula (III) in its isolated form is recorded after the storage period of step 2, using 1,2-dichloroethane as internal standard, in such a way that the molar ratio of the compound of formula (III) to the internal standard is 1:1. Value of integration of the peak assigned to the proton of the diiodomethyl radical is noted as A2.

Step 4: The rate of purity decrease, expressed as a percentage per day, is calculated as the result of $100 \times \frac{(A1-A2)}{l}$.

When the compound of formula (III) is such that X is $CO_2Me$ or $CO_2Et$, compound was found to be unstable upon storage, even when stored at - 30 °C.

As shown in figures 1, 2, 3 and 4, when the compound of formula (III) is such that X is adamantoyl, a rate of purity decrease lower than 1% per day has been measured over a period of 40 days in different storage conditions.

Example 2: Cyclopropanation of E-anethole

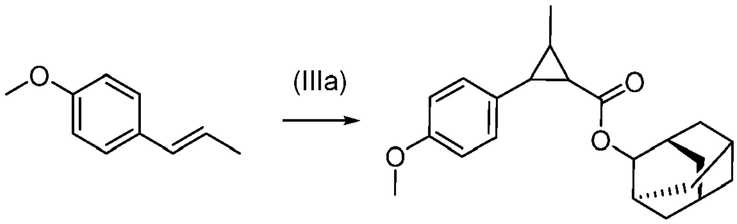


General Procedure D: To a 10 mL reaction tube equipped with a stirring bar was added adamantan-1-yl 2,2-diiodoacetate (IIIa) (45 mg, 0.10 mmol, 1 equiv) and the tube was sealed with a septum, degassed and filled with argon. Solvent (1ml), *E*-anethole (15 µL, 0.10 mmol, 1 equiv), the base (0.20 mmol, 2 equiv) and aqueous NaCl (1.25 M, 0.5 mL) were added. The reaction mixture was degassed by two freeze-pump-thaw cycles under argon. After that, the reaction tube was irradiated under the light source at a distance of 7 cm and a mini-fan was kept on top to maintain room temperature. After 18 h, the reaction mixture was passed through a short pad of silica gel and eluted with dichloromethane. The solvent was removed under *vacuum* and 1,2-dimethoxyethane (10 µL, 0.10 mmol,1 equiv) was added as internal standard. The mixture was then analysed by $^1$H-NMR in order to determine the yield of the reaction.

Table 2 below shows the results observed when several variations on protocol D were made:

Table 2

| Entry | Solvent | Base | Light source | Variations | Yield (%) |
|---|---|---|---|---|---|
| 1 | MeCN | $Et_3N$ | 21 W CFL | - | 41 |
| 2 | MeCN | i-$Pr_2NH$ | 21 W CFL | - | 17 |
| 3 | MeCN | DABCO | 21 W CFL | - | 11 |
| 4 | acetone | i-$Pr_2EtN$ | 21 W CFL | - | - |
| 5 | DCM | i-$Pr_2EtN$ | 21 W CFL | - | 16 |
| 6 | THF | i-$Pr_2EtN$ | 21 W CFL | - | 35 |
| 7 | MeCN | i-$Pr_2EtN$ | 21 W CFL | - | 60 |
| 8 | MeCN | none | 21 W CFL | - | 0 |
| 9 | MeCN | i-$Pr_2EtN$ | 21 W CFL | With 1 mol% $Ru(bpy)_3(PF_6)_2$ | 63 |
| 10 | MeCN | i-$Pr_2EtN$ | none | - | 0 |
| 11 | MeCN | i-$Pr_2EtN$ | 21 W CFL | Under air | 10 |
| 12 | MeCN | i-$Pr_2EtN$ | 21 W CFL | No addition of NaCl solution | 17 |
| 13 | MeCN | i-$Pr_2EtN$ | White LED | - | 42 |
| 14 | MeCN | i-$Pr_2EtN$ | Blue LED | - | 44 |

Entries 8, 9, 10 and 12 are provided as comparative examples. These comparative examples confirm that the

irradiation with light in combination with the presence of an electron donor and a biphasic system are essential features for the reaction to proceed efficiently. Entry 9 shows that the presence of a ruthenium based photosensitizer is not essential for the reaction to proceed efficiently.

Example 3: Scope of cyclopropanation reaction

General Procedure E: To a 10 mL reaction tube equipped with a stirring bar was added the compound of formula (III) as indicated in Table 3 (0.20 mmol, 1 equiv) and the tube was sealed with a septum, degassed and filled with argon. Acetonitrile (2 mL), substrate as indicated in Table 3 (0.20 mmol, 1 equiv), i-$Pr_2EtN$ (0.40 mmol, 2 equiv) and aqueous NaCl (1.25 M, 1 mL) were added. The reaction mixture was degassed by two freeze-pump-thaw cycles under argon. After that, the reaction tube was irradiated under the light source at a distance of 7 cm and a mini-fan was kept on top to maintain room temperature. After 3 hours, additional compound of formula (III) as indicated in Table 3 (0.20 mmol, 1 equiv) and i-$Pr_2EtN$ (0.40 mmol, 2 equiv) were added. After further 15 h, the reaction mixture was passed through a short pad of silica gel and eluted with dichloromethane. The solvent was removed under *vacuum* and 1,2-dimethoxyethane (10 μL, 0.20 mmol,1 equiv) was added as internal standard. The mixture was then analysed by $^1$H-NMR in order to determine the yield of the reaction.

General Procedure F: To a 10 mL reaction tube equipped with a stirring bar was added the compound of formula (III) as indicated in Table 3 (0.40 mmol, 2 equiv) and the tube was sealed with a septum, degassed and filled with argon. MeCN (2ml), the corresponding styrene (0.2 mmol, 1 equiv), *i*-$Pr_2EtN$ (0.40 mmol, 2 equiv) and aqueous NaCl (1.25 M, 1 mL) were added. The reaction mixture was degassed by two freeze-pump-thaw cycles under argon. After that, the reaction tube was irradiated under with visible light (21W CFL at a distance of 7 cm) and a mini-fan was kept on top to maintain room temperature. After 18 h, the reaction mixture was passed through a short pad of silica gel and eluted with dichloromethane. The solvent was removed under *vacuum* and the residue was purified by column chromatography on neutral silica gel to give the corresponding cyclopropanes. Ratio of isomers was determined from the crude reaction mixture using $^1$H NMR spectroscopy.

In this example, the compounds of formulae (IIIa), (IIIb), (IIIc), (IIId) and (IIIe) were used.

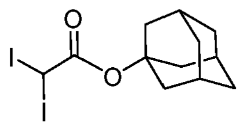
(IIIa)

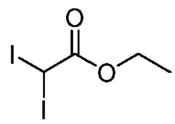
(IIIb)

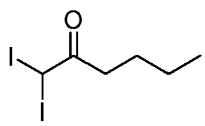
(IIIc)

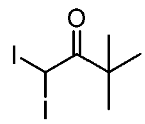
(IIId)

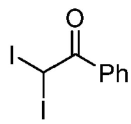
(IIIe)

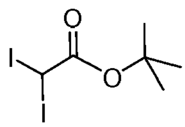
(IIIf)

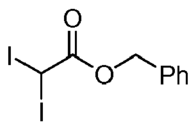
(IIIg)

Table 3 summarizes the obtained results:

Table 3

| Entry | Substrate | (III) | Product | Procedure | Yield (%) | cis:trans ratio |
|---|---|---|---|---|---|---|
| 1 | 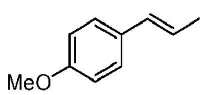 | (IIIa) | 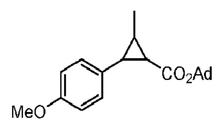 | E | 91 | 1.1:1 |
| 2 | | (IIIb) | 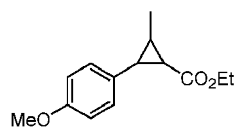 | E | 88 | 1:1 |

(continued)

| Entry | Substrate | (III) | Product | Procedure | Yield (%) | cis:trans ratio |
|---|---|---|---|---|---|---|
| 3 | 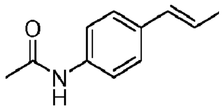 | (IIIa) | 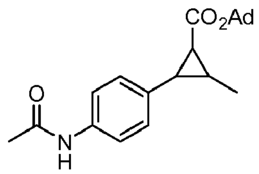 | E | 48 | 1:1 |
| 4 | 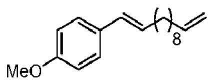 | (IIIb) | 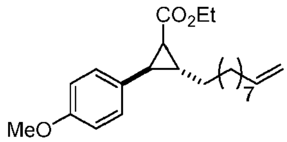 | F | 64 | 1:1 |
| 5 | 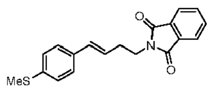 | (IIIa) | 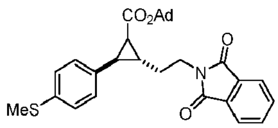 | E | 61 | 0.7:1 |
| 6 | 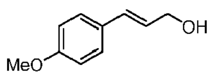 | (IIIa) | 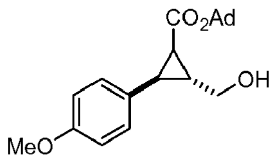 | E | 93 | 1:1 |
| 7 | 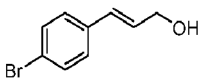 | (IIIa) | 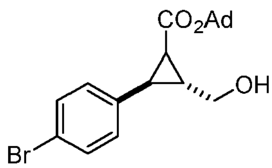 | F | 42 | 1:1 |
| 8 | 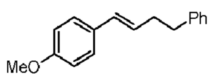 | (IIIa) | 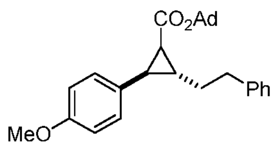 | F | 73 | 1:1 |
| 9 | 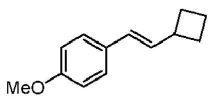 | (IIIa) | 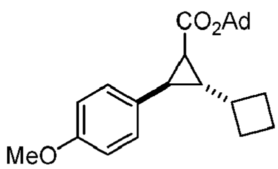 | E | 55 | 1:1 |
| 10 | 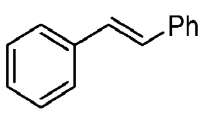 | (IIIb) | 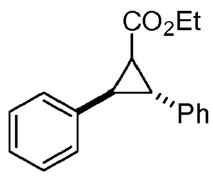 | F | 56 | 0:1 |
| 11 | 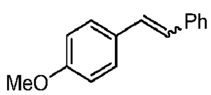 | (IIIa) | 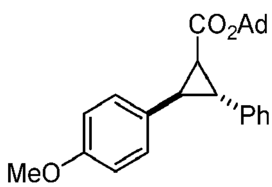 | E | 46 | 1:1 |

(continued)

| Entry | Substrate | (III) | Product | Procedure | Yield (%) | cis:trans ratio |
|---|---|---|---|---|---|---|
| 12 | 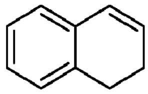 | (IIIb) | 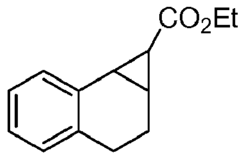 | F | 48 | - |
| 13 | 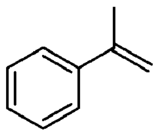 | (IIIa) | 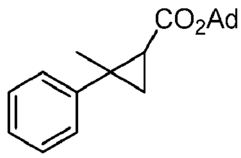 | F | 90 | 1:1 |
| 14 | 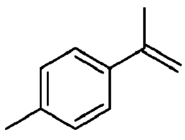 | (IIIb) | 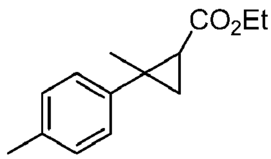 | E | 91 | 1:1 |
| 15 | 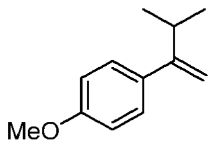 | (IIIa) | 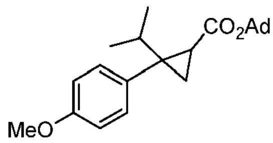 | F | 75 | 25:1 |
| 16 | 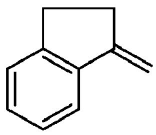 | (IIIb) | 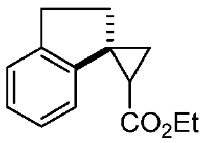 | F | 39 | 1:1 |
| 17 | 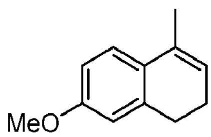 | (IIIa) | 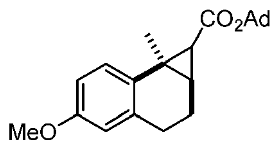 | F | 60 | 1:1 |
| 18 | 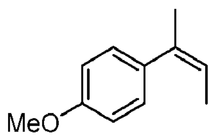 | (IIIa) | 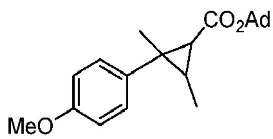 | E | 94 | 1:1 |
| 19 | 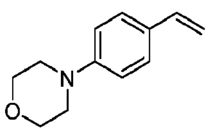 | (IIIa) | 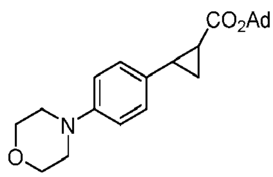 | E | 88 | 1:1 |
| 20 | 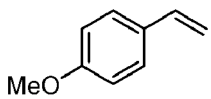 | (IIIa) | 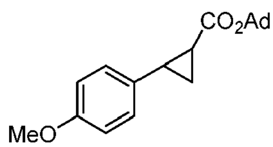 | E | 91 | 1:1 |
| 21 | 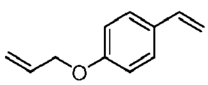 | (IIIa) | 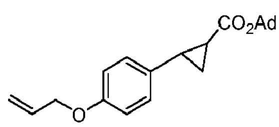 | E | 72 | 1:1 |

(continued)

| Entry | Substrate | (III) | Product | Procedure | Yield (%) | cis:trans ratio |
|---|---|---|---|---|---|---|
| 22 | 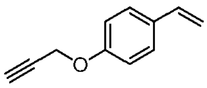 | (IIIa) | 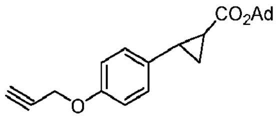 | F | 67 | 1:1 |
| 23 | 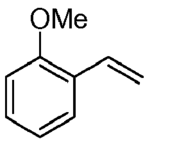 | (IIIa) | 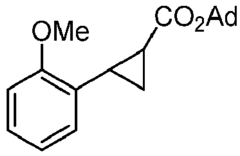 | F | 41 | 1:1 |
| 24 | 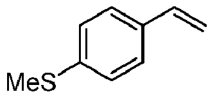 | (IIIa) | 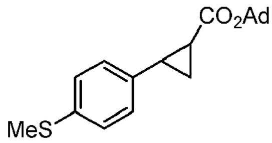 | E | 73 | 1:1 |
| 25 | 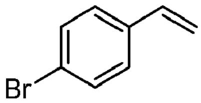 | (IIIb) | 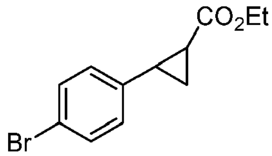 | F | 61 | 0.7:1 |
| 26 | 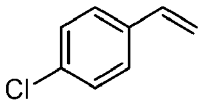 | (IIIb) | 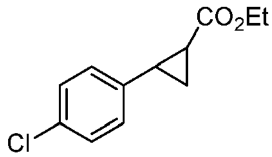 | F | 40 | 0.8:1 |
| 27 | 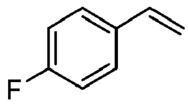 | (IIIb) | 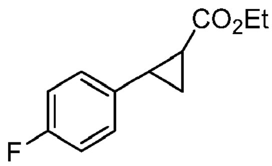 | F | 36 | 0.6:1 |
| 28 | 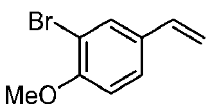 | (IIIa) | 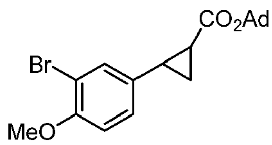 | E | 71 | 1:1 |
| 29 | 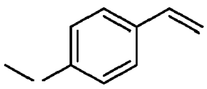 | (IIIb) | 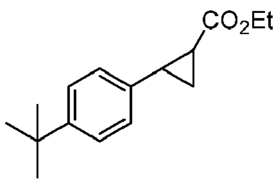 | E | 75 | 1:1 |
| 30 | 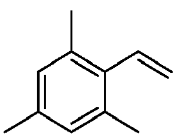 | (IIIb) | 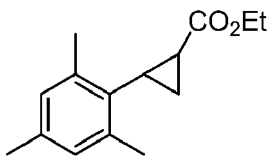 | F | 40 | 1.1:1 |

(continued)

| Entry | Substrate | (III) | Product | Procedure | Yield (%) | cis:trans ratio |
|---|---|---|---|---|---|---|
| 31 | 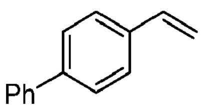 | (IIIb) | 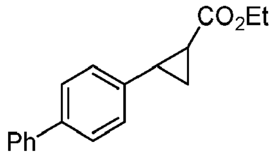 | E | 69 | 2:3 |
| 32 | 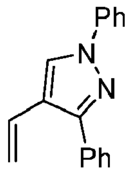 | (IIIa) | 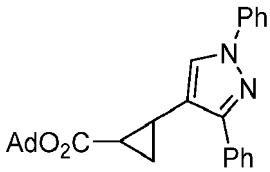 | F | 73 | 2:1 |
| 33 | 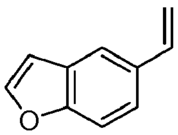 | (IIIa) | 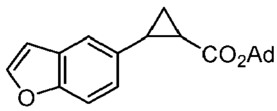 | F | 90 | 1:1 |
| 34 | 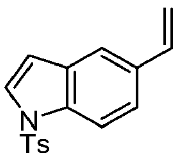 | (IIIa) | 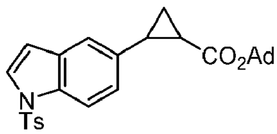 | F | 58 | 1:1 |
| 35 | 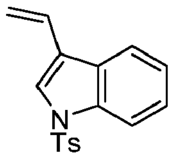 | (IIIa) | 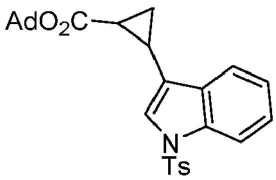 | F | 35 | 3:1 |
| 36 | 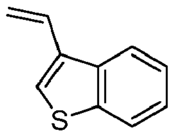 | (IIIa) | 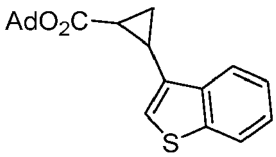 | F | 38 | 1:1 |
| 37 | 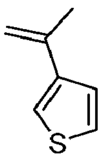 | (IIIa) | 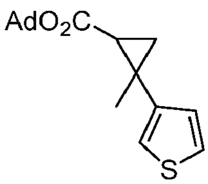 | F | 71 | 1:1 |
| 38 | 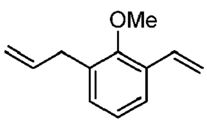 | (IIIa) | 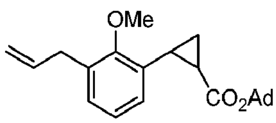 | F | 62 | 1:1 |
| 39 | 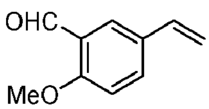 | (IIIa) | 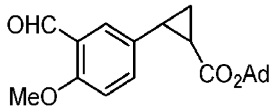 | F | 56 | 1:0.7 |
| 40 | 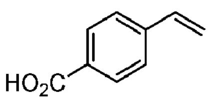 | (IIIa) | 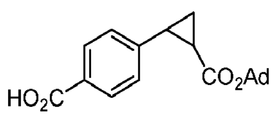 | F | 38 | 1:1 |

(continued)

| Entry | Substrate | (III) | Product | Procedure | Yield (%) | cis:trans ratio |
|---|---|---|---|---|---|---|
| 41 | 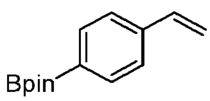 | (IIIa) | 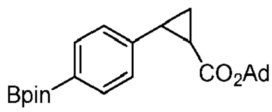 | F | 35 | 1:1 |
| 42 | 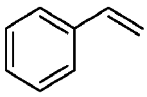 | (IIIb) | 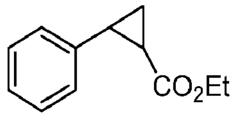 | F | 67 | 1:1 |
| 43 | 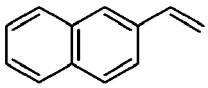 | (IIIb) | 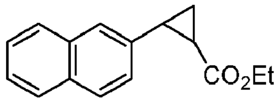 | F | 57 | 1:3 |
| 44 | 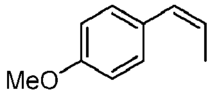 | (IIIc) | 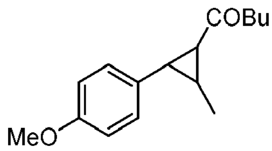 | E | 33 | 2:3 |
| 45 | 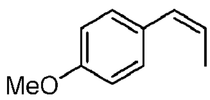 | (IIId) | 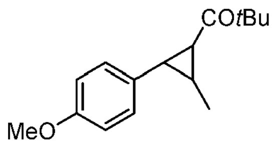 | E | 49 | 2:3 |
| 46 | 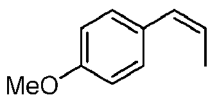 | (IIIe) | 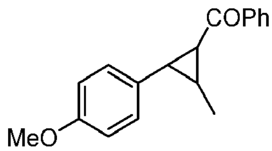 | F | 47 | 0.8:1 |
| 47 | 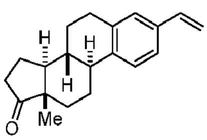 | (IIIa) | 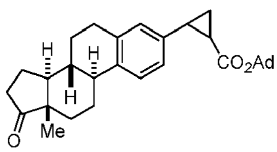 | E | 50 | 1:1 |
| 48 | 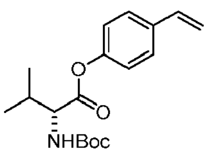 | (IIIa) | 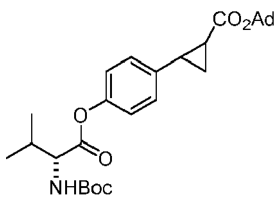 | E | 61 | 1:1 |
| 49 | 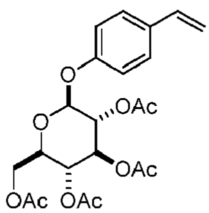 | (IIIa) | 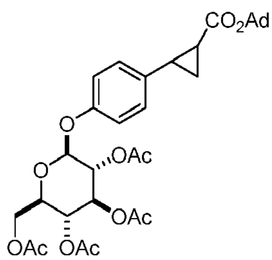 | E | 62 | 1:1 |

(continued)

| Entry | Substrate | (III) | Product | Procedure | Yield (%) | cis:trans ratio |
|---|---|---|---|---|---|---|
| 50 | 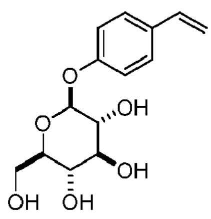 | (IIIa) | 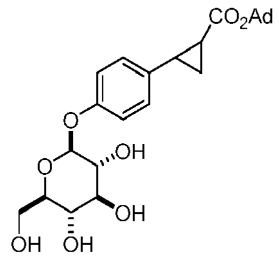 | E | 30 | 1:1 |
| 51 | 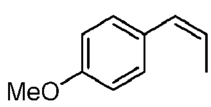 | (IIIf) | 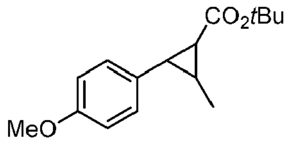 | E | 63 | - |
| 52 | 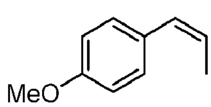 | (IIIg) | 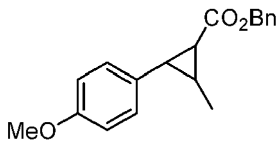 | E | 71 | - |

Entries 1-52 of Example 3 indicate that the method of the first aspect of the invention is robust and can be carried out on a broad scope of compounds comprising a fragment of formula (II), even when said compounds bear other functional groups such as hydroxyl (entry 50), formyl (entry 39), carboxyl (entry 40), and amide (entry 3). In particular, when the compound comprising a fragment of formula (II) further comprises unsaturated carbon-carbon bonds (entries 4, 21, 22, 38), the method of the invention is highly chemoselective for the double bond comprised in the fragment of formula (II).

**CITATION LIST**

1. del Hoyo, A.; Garcia-Herraiz, A.; Garcia-Suero, M. Angew. Chem. Int. Ed. 2017, 56, 1610 -1613.
2. del Hoyo, A.; Garcia-Suero, M. Eur. J. Org. Chem. 2017, 2122-2125.
3. Li, P.; Zhao, J.; Shi, L.; Wang, J.; Shi, X.; Li, F. Nature Communications, 2018, 9:1972 - DOI: 10.1038/s41467-018-04331-4.
4. Sayes, M., Benoit, G.; Charette, A.B. Angew. Chem. Int. Ed. 2018, 57, 13514 - 13518.
5. Phelan, J.P.; Lang, S.B.; Compton, J.S.; Kelly, C.B. ; Dykstra, R.; Gutierrez, O.; Molander, G.A. J. Am. Chem. Soc. 2018, 140, 8037-8047.

## Claims

1. A method for the preparation of a compound comprising a fragment of formula (I) from a compound comprising a fragment of formula (II)

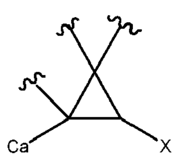

(I)

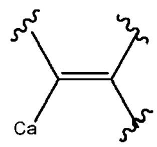

(II)

wherein the method converts the fragment of formula (II) into the fragment of formula (I), $C_a$ is a carbon atom that

is member of an aromatic or heteroaromatic ring system, each wavy line represents a connection to the remaining parts of the compound comprising the fragment of formula (I) or of formula (II), X is a radical selected from the group consisting of a radical of formula $-COR_1$ and a radical of formula $-COOR_1$, being $R_1$ a radical selected from the group consisting of hydrogen, a $(C_1-C_{12})$alkyl radical a benzyl radical and a radical deriving from a ring system comprising from 1 to 4 bridged or fused rings, each ring comprising from 3 to 6 members and being saturated or aromatic, the members of the rings being selected from the group consisting of C, CH, $CH_2$, O, S, N and NH and wherein each ring is further optionally substituted at any available position with a radical selected from the group consisting of nitro, halo, cyano, $(C_1-C_{12})$haloalkyl, $(C_1-C_{12})$alkyloxy, $(C_1-C_{12})$alkylcarbonyl, $(C_1-C_{12})$alkyloxycarbonyl and $(C_1-C_{12})$alkylcarbonyloxy, said method comprising the step of contacting the compound comprising the fragment of formula (II) with a compound of formula (III)

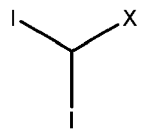

(III)

under light irradiation and in the presence of an electron donor and of a biphasic solvent system consisting of an organic phase and an aqueous phase.

**2.** The method according to claim 1 that is carried out under an inert atmosphere.

**3.** The method according to any of claims 1 and 2 wherein the fragment of formula (II) is a fragment of formula (II')

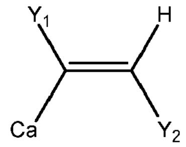

(II')

wherein $Y_1$ and $Y_2$ independently represent a hydrogen atom or a carbon atom through which the molecular fragment of formula (II) is connected to the remainder parts of the compound comprising the fragment of formula (II).

**4.** The method according to any of claims 1 to 3 wherein the electron donor is selected from the group consisting of a cathode, a photo-cathode and a compound of formula $NR_2R_3R_4$ wherein each of $R_2$, $R_3$ and $R_4$ is a $(C_1-C_6)$alkyl group, equal or different.

**5.** The method according to any of claims 1 to 4 wherein the electron donor is diisopropylethylamine; preferably wherein the molar ratio of diisopropylethylamine to the compound comprising the fragment of formula (II) is 5n:n, being n the number of fragments of formula (II) in the compound comprising the fragment of formula (II).

**6.** The method according to any of claims 1 to 5 wherein the biphasic solvent system consisting of an organic phase and an aqueous phase is such that the organic phase is a solvent selected from the group consisting of acetonitrile, tetrahydrofuran, 1,4-dioxane, diethyl ether, methyl-*tert*-butylether, dichloromethane, dichloroethane, chloroform and tetrachloroethane, and the aqueous phase is selected from water and an aqueous solution of a water soluble salt in a suitable concentration for the solvent system to be biphasic;

**7.** The method according to any of claims 1 to 6 wherein the biphasic solvent system consisting of an organic phase and an aqueous phase is such that the organic phase is acetonitrile and the aqueous phase is an aqueous solution of a sodium chloride in a suitable concentration for the solvent system to be biphasic.

**8.** The method according to any of claims 1 to 7 wherein the light irradiation is carried out with a compact fluorescent lamp; preferably of at least 20 W potency.

**9.** The method according to any of claims 1 to 8 wherein the molar ratio of the compound of formula (III) to the compound comprising the fragment of formula (II) is 2n:n, being n the number of fragments of formula (II) in the compound

comprising the fragment of formula (II).

**10.** The method according to any of claims 1 to 9 wherein X is selected from the group consisting of a radical of formula $-COR_1$, and a radical of formula $-COOR_1$ being $R_1$ a radical selected from the group consisting of a $(C_1-C_{12})$alkyl radical, a benzyl radical, a $(C_6-C_{20})$aryl radical and a radical deriving from a saturated hydrocarbon ring system comprising from 1 to 4 bridged or fused rings, each ring comprising from 3 to 6 members; preferably wherein $R_1$ is selected from the group consisting of ethyl, phenyl, adamantyl, butyl, benzyl and *tert*-butyl.

**11.** A compound of formula (III')

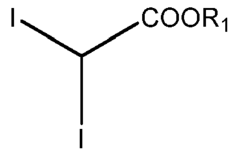

(III')

that is stable in its isolated form, wherein $R_1$ is as defined in claim 1 and the term "stable in its isolated form" refers to the fact that the purity of the isolated compound does not decrease at a rate higher than one percent per day upon storage under air, at room temperature and ambient light irradiation.

**12.** The compound according to claim 11 wherein $R_1$ is as defined in claim 10.

**13.** The compound according to any of claims 11 and 12 that is a solid in standard conditions of pressure and temperature.

**14.** The compound according to any of claims 11 to 13 of formula (IIIa)

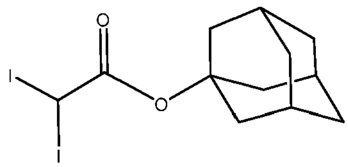

(IIIa)

**15.** Use of the compound according to any of claims 11 to 14 as a reagent for cyclopropanation reactions.

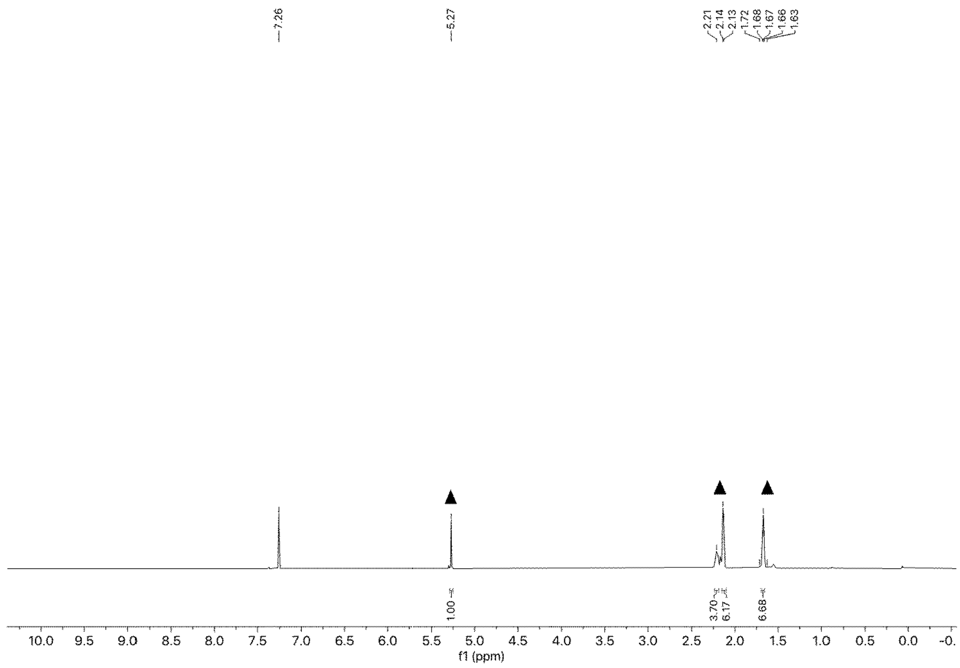

FIGURE 1

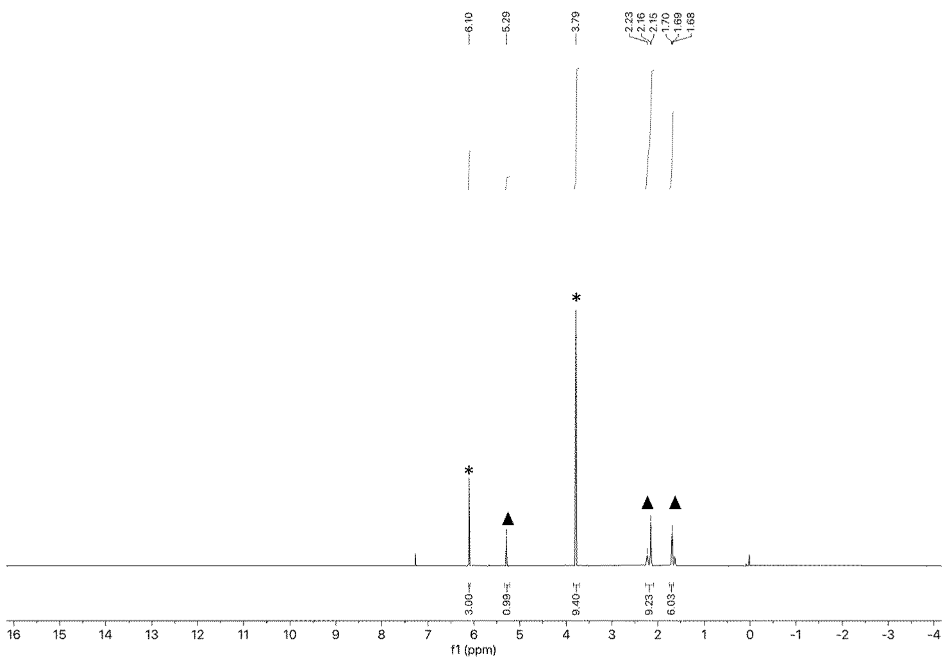

FIGURE 2

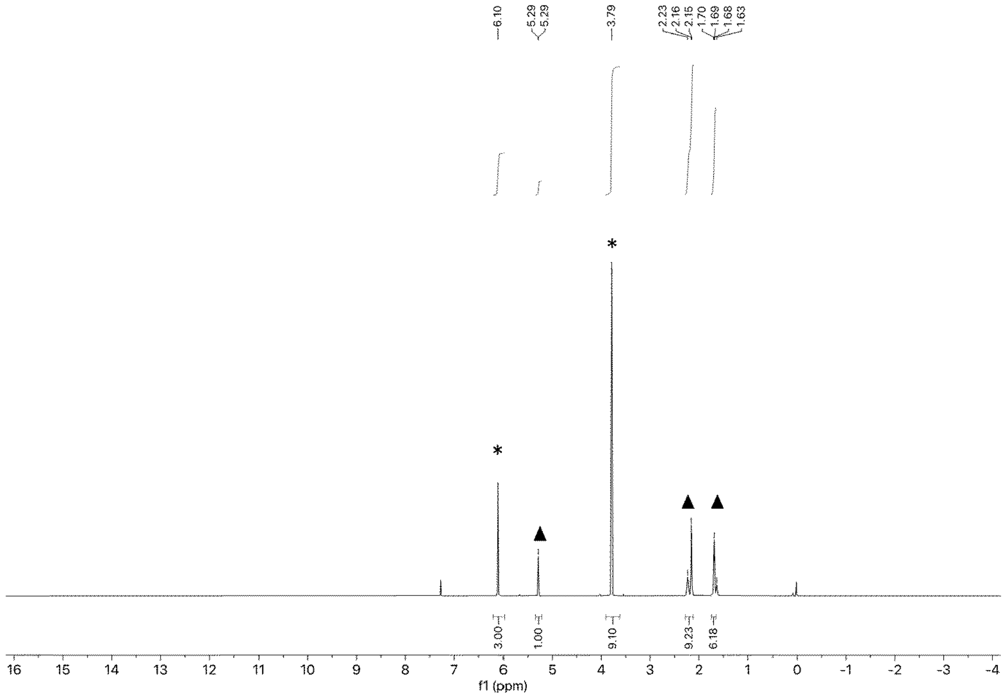

FIGURE 3

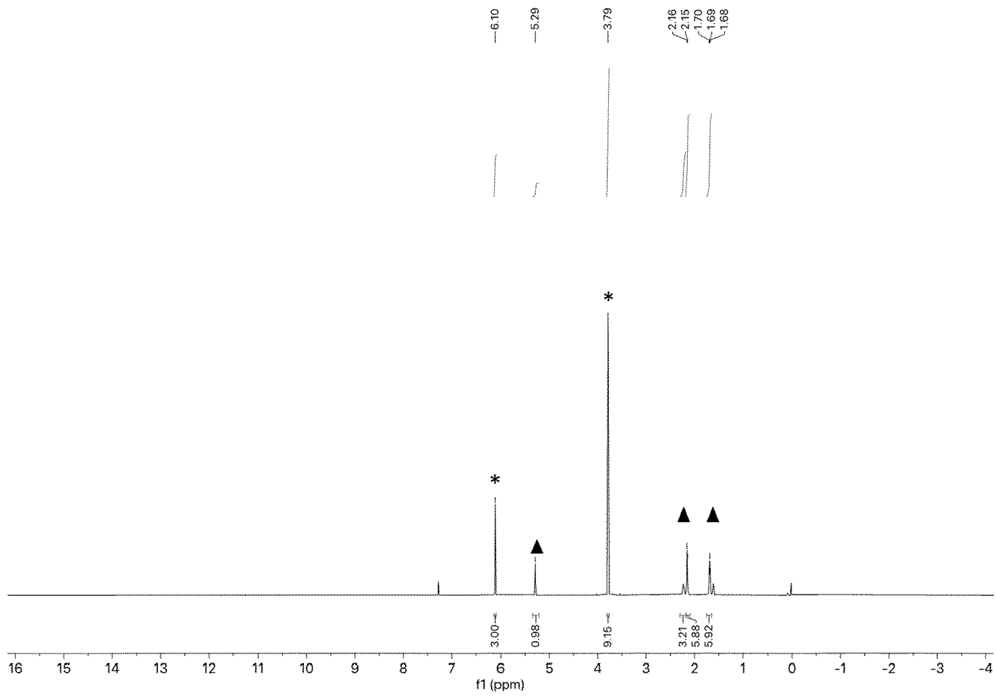

FIGURE 4

Europäisches Patentamt
European Patent Office
Office européen des brevets

# EUROPEAN SEARCH REPORT

Application Number
EP 19 38 2720

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LEBEL: "Stereoselective cyclopropanation reactions", CHEMICAL REVIEWS, vol. 103, no. 4, 1 January 2003 (2003-01-01), page 977, XP055059894, ISSN: 0009-2665, DOI: 10.1021/cr010007e | 11-13,15 | INV. C07C67/313 C07C69/743 |
| A | * page 999 * | 1-10,14 | |
| X | R L COBB: "Synthesis and Properties of Triiodoacetic Acid and Its Salts", JOURNAL OF ORGANIC CHEMISTRY, vol. 23, 1 January 1958 (1958-01-01), pages 1368-1369, XP55671345, DOI: 10.1021/jo01103a038 | 11-13 | |
| A | * page 1368, first column * | 1-10,14, 15 | |
| X | VICTOR L. HEASLEY ET AL: "Reactions of terminal alkynes with iodine in methanol", JOURNAL OF ORGANIC CHEMISTRY, vol. 45, no. 23, 1 November 1980 (1980-11-01), pages 4649-4652, XP55671348, US ISSN: 0022-3263, DOI: 10.1021/jo01311a020 | 11-13 | TECHNICAL FIELDS SEARCHED (IPC) C07C |
| A | * compounds 5a-5c * | 1-10,14, 15 | |

The present search report has been drawn up for all claims

1

| Place of search | Date of completion of the search | Examiner |
|---|---|---|
| The Hague | 24 February 2020 | Hacking, Michiel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **DEL HOYO.** *Angew. Chem. Int. Ed.,* 2017, vol. 56, 1610-1613
- *Eur. J. Org. Chem.,* 2017, 2122-2125
- **LI.** *Nature Communications,* 2018, vol. 9, 1972
- *Angew. Chem. Int. Ed.,* 2018, vol. 57, 13514-13518
- **PHELAN.** *J. Am. Chem. Soc.,* 2018, vol. 140, 8037-8047
- **DEL HOYO, A. ; GARCIA-HERRAIZ, A. ; GARCIA-SUERO, M.** *Angew. Chem. Int. Ed.,* 2017, vol. 56, 1610-1613
- **DEL HOYO, A. ; GARCIA-SUERO, M.** *Eur. J. Org. Chem.,* 2017, 2122-2125
- **LI, P. ; ZHAO, J. ; SHI, L. ; WANG, J. ; SHI, X. ; LI, F.** *Nature Communications,* 2018, vol. 9, 1972
- **SAYES, M. ; BENOIT, G. ; CHARETTE, A.B.** *Angew. Chem. Int. Ed.,* 2018, vol. 57, 13514-13518
- **PHELAN, J.P. ; LANG, S.B. ; COMPTON, J.S. ; KELLY, C.B. ; DYKSTRA, R. ; GUTIERREZ, O. ; MOLANDER, G.A.** *J. Am. Chem. Soc.,* 2018, vol. 140, 8037-8047